# EUROPÄISCHE PATENTSCHRIFT

(11) **EP 2 129 786 B1**
(45) Veröffentlichungstag und Bekanntmachung des Hinweises auf die Patenterteilung: **20.08.2014**
(21) Anmeldenummer: 08708980.1
(22) Anmeldetag: 14.02.2008
(51) Int. Cl.: C12P 13/04, C12N 15/00, C12R 1/01

(54) **VERFAHREN ZUR HERSTELLUNG VON L-AMINOSÄUREN UNTER VERWENDUNG VON STÄMMEN DER FAMILIE ENTEROBACTERIACEAE**
METHOD FOR THE PRODUCTION OF L-AMINO ACIDS USING STRAINS OF THE ENTEROBACTERIACEAE<I></I>FAMILY
PROCÉDÉ DE FABRICATION DE L-AMINOACIDES AU MOYEN DE SOUCHES DE LA FAMILLE DES ENTÉROBACTÉRIACÉES

(30) Priorität: 05.03.2007 DE 102007010519; 25.10.2007 DE 102007051024
(43) Veröffentlichungstag der Anmeldung: 09.12.2009
(73) Patentinhaber: Evonik Degussa GmbH, 45128 Essen (DE)
(72) Erfinder: RIEPING, Mechthild, 33619 Bielefeld (DE); THIERBACH, Georg, 33613 Bielefeld (DE); KREUTZER, Caroline, 33813 Oerlinghausen (DE)
(86) Internationale Anmeldenummer: PCT/EP2008/051780
(87) Internationale Veröffentlichungsnummer: WO 2008/107277

(56) Entgegenhaltungen:
- EP-A- 1 715 055
- WO-A1-2008/081959
- HONISCH C ET AL: "High-troughput mutation detection underlying adaptive evolution of Escherichia coli-K12" GENOME RESEARCH, COLD SPRING HARBOR LABORATORY PRESS, WOODBURY, NY, US, Bd. 14, Nr. 12, 1. Dezember 2004 (2004-12-01), Seiten 2495-2502, XP002991060 ISSN: 1088-9051
- HERRING CHRISTOPHER D ET AL: "Comparative genome sequencing of Escherichia coli allows observation of bacterial evolution on a laboratory timescale" NATURE GENETICS, NATURE AMERICA, NEW YORK, US, Bd. 38, Nr. 12, 1. Dezember 2006 (2006-12-01), Seiten 1406-1412, XP002473519 ISSN: 1061-4036
- ORMO MATS ET AL: "Crystal structure of a complex of Escherichia coli glycerol kinase and an allosteric effector fructose 1,6-bisphosphate" BIOCHEMISTRY, AMERICAN CHEMICAL SOCIETY. EASTON, PA, US, Bd. 37, Nr. 47, 24. November 1998 (1998-11-24), Seiten 16565-16572, XP002473518 ISSN: 0006-2960
- HOLTMAN C KAY ET AL: "Reverse genetics of Escherichia coli glycerol kinase allosteric regulation and glucose control of glycerol utilization in vivo" JOURNAL OF BACTERIOLOGY, WASHINGTON, DC, US, Bd. 183, Nr. 11, 1. Juni 2001 (2001-06-01), Seiten 3336-3344, XP002473520 ISSN: 0021-9193
- LIN E C C ED - NEIDHARDT F C ET AL: "DISSIMILATORY PATHWAYS FOR SUGARS, POLYOLS, AND CARBOXYLATES" ESCHERICHIA COLI AND SALMONELLA. CELLULAR AND MOLECULAR BIOLOGY, WASHINGTON, ASM PRESS, US, 1. Januar 1996 (1996-01-01), Seiten 307-342, XP001246975 ISBN: 978-1-55581-084-9

SEQUENCE LISTING
<110> Evonik Degussa GmbH
<120> Verfahren zur Herstellung von L-Aminosäuren unter Verwendung verbesserter Stämme der Familie Enterobacteriaceae
<130> 2007P00111
<160> 20
<170> PatentIn version 3.3
<210> 1
   <211> 1509
   <212> DNA
   <213> Escherichia coli
<220>
   <221> CDS
   <222> (1)..(1509)
   <223> glpK Kodierregion
<400> 1
   atg act gaa aaa aaa tat atc gtt gcg ctc gac cag ggc acc acc agc      48
   Met Thr Glu Lys Lys Tyr Ile Val Ala Leu Asp Gln Gly Thr Thr Ser
   1               5                   10                  15
   tcc cgc gcg gtc gta atg gat cac gat gcc aat atc att agc gtg tcg      96
   Ser Arg Ala Val Val Met Asp His Asp Ala Asn Ile Ile Ser Val Ser
               20                  25                  30
   cag cgc gaa ttt gag caa atc tac cca aaa cca ggt tgg gta gaa cac     144
   Gln Arg Glu Phe Glu Gln Ile Tyr Pro Lys Pro Gly Trp Val Glu His
           35                  40                  45
   gac cca atg gaa atc tgg gcc acc caa agc tcc acg ctg gta gaa gtg     192
   Asp Pro Met Glu Ile Trp Ala Thr Gln Ser Ser Thr Leu Val Glu Val
       50                  55                  60
   ctg gcg aaa gcc gat atc agt tcc gat caa att gca gct atc ggt att     240
   Leu Ala Lys Ala Asp Ile Ser Ser Asp Gln Ile Ala Ala Ile Gly Ile
   65                  70                  75                  80
   acg aac cag cgt gaa acc act att gtc tgg gaa aaa gaa acc ggc aag     288
   Thr Asn Gln Arg Glu Thr Thr Ile Val Trp Glu Lys Glu Thr Gly Lys
                   85                  90                  95
   cct atc tat aac gcc att gtc tgg cag tgc cgt cgt acc gca gaa atc     336
   Pro Ile Tyr Asn Ala Ile Val Trp Gln Cys Arg Arg Thr Ala Glu Ile
               100                 105                 110
   tgc gag cat tta aaa cgt gac ggt tta gaa gat tat atc cgc agc aat     384
   Cys Glu His Leu Lys Arg Asp Gly Leu Glu Asp Tyr Ile Arg Ser Asn
           115                 120                 125
   acc ggt ctg gtg att gac ccg tac ttt tct ggc acc aaa gtg aag tgg     432
   Thr Gly Leu Val Ile Asp Pro Tyr Phe Ser Gly Thr Lys Val Lys Trp
       130                 135                 140
   atc ctc gac cat gtg gaa ggc tct cgc gag cgt gca cgt cgt ggt gaa     480
   Ile Leu Asp His Val Glu Gly Ser Arg Glu Arg Ala Arg Arg Gly Glu
   145                 150                 155                 160
   ttg ctg ttt ggt acg gtt gat acg tgg ctt atc tgg aaa atg act cag     528
   Leu Leu Phe Gly Thr Val Asp Thr Trp Leu Ile Trp Lys Met Thr Gln
                   165                 170                 175
   ggc cgt gtc cat gtg acc gat tac acc aac gcc tct cgt acc atg ttg         576
   Gly Arg Val His Val Thr Asp Tyr Thr Asn Ala Ser Arg Thr Met Leu
               180                 185                 190
   ttc aac atc cat acc ctg gac tgg gac gac aaa atg ctg gaa gtg ctg         624
   Phe Asn Ile His Thr Leu Asp Trp Asp Asp Lys Met Leu Glu Val Leu
           195                 200                 205
   gat att ccg cgc gag atg ctg cca gaa gtg cgt cgt tct tcc gaa gta         672
   Asp Ile Pro Arg Glu Met Leu Pro Glu Val Arg Arg Ser Ser Glu Val
       210                 215                 220
   tac ggt cag act aac att ggc ggc aaa ggc ggc acg cgt att cca atc         720
   Tyr Gly Gln Thr Asn Ile Gly Gly Lys Gly Gly Thr Arg Ile Pro Ile
   225                 230                 235                     240
   tcc ggg atc gcc ggt gac cag cag gcc gcg ctg ttt ggt cag ttg tgc         768
   Ser Gly Ile Ala Gly Asp Gln Gln Ala Ala Leu Phe Gly Gln Leu Cys
                   245                 250                 255
   gtg aaa gaa ggg atg gcg aag aac acc tat ggc act ggc tgc ttt atg         816
   Val Lys Glu Gly Met Ala Lys Asn Thr Tyr Gly Thr Gly Cys Phe Met
               260                 265                 270
   ctg atg aac act ggc gag aaa gcg gtg aaa tca gaa aac ggc ctg ctg         864
   Leu Met Asn Thr Gly Glu Lys Ala Val Lys Ser Glu Asn Gly Leu Leu
           275                 280                 285
   acc acc atc gcc tgc ggc ccg act ggc gaa gtg aac tat gcg ttg gaa         912
   Thr Thr Ile Ala Cys Gly Pro Thr Gly Glu Val Asn Tyr Ala Leu Glu
       290                 295                 300
   ggt gcg gtg ttt atg gca ggc gca tcc att cag tgg ctg cgc gat gaa         960
   Gly Ala Val Phe Met Ala Gly Ala Ser Ile Gln Trp Leu Arg Asp Glu
   305                 310                 315                     320
   atg aag ttg att aac gac gcc tac gat tcc gaa tat ttc gcc acc aaa        1008
   Met Lys Leu Ile Asn Asp Ala Tyr Asp Ser Glu Tyr Phe Ala Thr Lys
                   325                 330                 335
   gtg caa aac acc aat ggt gtg tat gtg gtt ccg gca ttt acc ggg ctg        1056
   Val Gln Asn Thr Asn Gly Val Tyr Val Val Pro Ala Phe Thr Gly Leu
               340                 345                 350
   ggt gcg ccg tac tgg gac ccg tat gcg cgc ggg gcg att ttc ggt ctg        1104
   Gly Ala Pro Tyr Trp Asp Pro Tyr Ala Arg Gly Ala Ile Phe Gly Leu
           355                 360                 365
   act cgt ggg gtg aac gct aac cac att ata cgc gcg acg ctg gag tct        1152
   Thr Arg Gly Val Asn Ala Asn His Ile Ile Arg Ala Thr Leu Glu Ser
       370                 375                 380
   att gct tat cag acg cgt gac gtg ctg gaa gcg atg cag gcc gac tct        1200
   Ile Ala Tyr Gln Thr Arg Asp Val Leu Glu Ala Met Gln Ala Asp Ser
   385                 390                 395                     400
   ggt atc cgt ctg cac gcc ctg cgc gtg gat ggt ggc gca gta gca aac        1248
   Gly Ile Arg Leu His Ala Leu Arg Val Asp Gly Gly Ala Val Ala Asn
                   405                 410                 415
   aat ttc ctg atg cag ttc cag tcc gat att ctc ggc acc cgc gtt gag        1296
   Asn Phe Leu Met Gln Phe Gln Ser Asp Ile Leu Gly Thr Arg Val Glu
               420                 425                 430
   cgc ccg gaa gtg cgc gaa gtc acc gca ttg ggt gcg gcc tat ctc gca        1344
   Arg Pro Glu Val Arg Glu Val Thr Ala Leu Gly Ala Ala Tyr Leu Ala
           435                 440                 445
   ggc ctg gcg gtt ggc ttc tgg cag aac ctc gac gag ctg caa gag aaa        1392
   Gly Leu Ala Val Gly Phe Trp Gln Asn Leu Asp Glu Leu Gln Glu Lys
       450                 455                 460
   gcg gtg att gag cgc gag ttc cgt cca ggc atc gaa acc act gag cgt      1440
   Ala Val Ile Glu Arg Glu Phe Arg Pro Gly Ile Glu Thr Thr Glu Arg
   465                 470                 475                 480
   aat tac cgt tac gca ggc tgg aaa aaa gcg gtt aaa cgc gcg atg gcg      1488
   Asn Tyr Arg Tyr Ala Gly Trp Lys Lys Ala Val Lys Arg Ala Met Ala
                   485                 490                 495
   tgg gaa gaa cac gac gaa taa                                          1509
   Trp Glu Glu His Asp Glu
               500
<210> 3
   <211> 1509
   <212> DNA
   <213> Salmonella typhimurium
<220>
   <221> CDS
   <222> (1)..(1509)
   <223> glpK Kodierregion
<400> 3
   atg act gaa aaa aaa tat atc gtt gcg ctc gac cag ggt acc acc agc       48
   Met Thr Glu Lys Lys Tyr Ile Val Ala Leu Asp Gln Gly Thr Thr Ser
   1               5                   10                  15
   tcc cgc gcg gtc gtt atg gat cat gac gcg aat atc gtc agc gta tca       96
   Ser Arg Ala Val Val Met Asp His Asp Ala Asn Ile Val Ser Val Ser
               20                  25                  30
   cag cgt gaa ttt gag caa att tat cct aag cca ggc tgg gta gaa cac      144
   Gln Arg Glu Phe Glu Gln Ile Tyr Pro Lys Pro Gly Trp Val Glu His
           35                  40                  45
   gac ccg atg gaa att tgg gcg tcc caa agc tca acg ctg gta gaa gtg      192
   Asp Pro Met Glu Ile Trp Ala Ser Gln Ser Ser Thr Leu Val Glu Val
       50                  55                  60
   ctg gct aag gcc gat atc agt tcc gat cag att gcc gct atc ggt atc      240
   Leu Ala Lys Ala Asp Ile Ser Ser Asp Gln Ile Ala Ala Ile Gly Ile
   65                  70                  75                  80
   acc aac caa cgt gaa acc gcg atc gtc tgg gaa cgt gaa acc ggc aaa      288
   Thr Asn Gln Arg Glu Thr Ala Ile Val Trp Glu Arg Glu Thr Gly Lys
                   85                  90                  95
   ccg ata tac aac gct att gtc tgg cag tgc cgt cgt acc gcc gat att      336
   Pro Ile Tyr Asn Ala Ile Val Trp Gln Cys Arg Arg Thr Ala Asp Ile
               100                 105                 110
   tgc gaa cag ctt aaa cgc gac ggc ctg gaa gat tac atc cgc gac aat      384
   Cys Glu Gln Leu Lys Arg Asp Gly Leu Glu Asp Tyr Ile Arg Asp Asn
           115                 120                 125
   acc ggt ctg gtc gtc gac ccg tac ttc tct ggc act aaa gtg aaa tgg      432
   Thr Gly Leu Val Val Asp Pro Tyr Phe Ser Gly Thr Lys Val Lys Trp
       130                 135                 140
   atc ctt gac cat gtg gaa ggt tcg cgc gaa cgc gcg aag cgt ggc gag      480
   Ile Leu Asp His Val Glu Gly Ser Arg Glu Arg Ala Lys Arg Gly Glu
   145                 150                 155                 160
   ctg ctg ttc ggt acg gtg gat acc tgg ctt atc tgg aag atg acg cag      528
   Leu Leu Phe Gly Thr Val Asp Thr Trp Leu Ile Trp Lys Met Thr Gln
                   165                 170                 175
   ggc cgc gtc cac gtt acg gat tac acc aac gcc tca cga acc atg ctg      576
   Gly Arg Val His Val Thr Asp Tyr Thr Asn Ala Ser Arg Thr Met Leu
               180                 185                 190
   ttc aac atc cat gat ttg gac tgg gac gat aag atg ctg gac gtg ctg      624
   Phe Asn Ile His Asp Leu Asp Trp Asp Asp Lys Met Leu Asp Val Leu
           195                 200                 205
   gat atc cca cgc gcg atg ctg ccc cag gta cgt aag tcc tcc gaa gtc      672
   Asp Ile Pro Arg Ala Met Leu Pro Gln Val Arg Lys Ser Ser Glu Val
       210                 215                 220
   tac ggc cag acc aat att ggc ggt aaa ggc ggt aca cgt att cct atc      720
   Tyr Gly Gln Thr Asn Ile Gly Gly Lys Gly Gly Thr Arg Ile Pro Ile
   225                 230                 235                 240
   gcc ggg att gcc ggg gat cag cag gcc gcg tta ttc ggt caa ctg tgc      768
   Ala Gly Ile Ala Gly Asp Gln Gln Ala Ala Leu Phe Gly Gln Leu Cys
                   245                 250                 255
   gta aag gaa gga atg gca aag aat acc tac ggc acc ggc tgc ttt atg      816
   Val Lys Glu Gly Met Ala Lys Asn Thr Tyr Gly Thr Gly Cys Phe Met
               260                 265                 270
   ctg atg aac acc ggc gag aaa gcg gta aaa tcg gaa aac ggc ctg ctg      864
   Leu Met Asn Thr Gly Glu Lys Ala Val Lys Ser Glu Asn Gly Leu Leu
           275                 280                 285
   atc acc atc gcc tgc ggc cca agc ggc gaa gtc aac tat gcg ctg gaa      912
   Ile Thr Ile Ala Cys Gly Pro Ser Gly Glu Val Asn Tyr Ala Leu Glu
       290                 295                 300
   ggc gcg gtg ttt atg gcg gga gcg tcc att caa tgg ctg cgt gat gaa      960
   Gly Ala Val Phe Met Ala Gly Ala Ser Ile Gln Trp Leu Arg Asp Glu
   305                 310                 315                 320
   atg aag ctc atc agc gac gcg ttc gac tcc gaa tat ttc gcc acg aaa     1008
   Met Lys Leu Ile Ser Asp Ala Phe Asp Ser Glu Tyr Phe Ala Thr Lys
                   325                 330                 335
   gtg aaa gat acc aac ggt gtc tac gtg gtt ccg gcc ttc acc ggt ctg     1056
   Val Lys Asp Thr Asn Gly Val Tyr Val Val Pro Ala Phe Thr Gly Leu
               340                 345                 350
   ggc gcg ccg tac tgg gac ccg tat gcc cgc ggc gcc att ttt ggc ctg     1104
   Gly Ala Pro Tyr Trp Asp Pro Tyr Ala Arg Gly Ala Ile Phe Gly Leu
           355                 360                 365
   acc cgc ggc gtg aac tct aat cac att atc cgc gcg acg ctc gaa tcc     1152
   Thr Arg Gly Val Asn Ser Asn His Ile Ile Arg Ala Thr Leu Glu Ser
       370                 375                 380
   atc gct tac cag acc cgc gac gta ctg gaa gcg atg cag gcc gac tcc     1200
   Ile Ala Tyr Gln Thr Arg Asp Val Leu Glu Ala Met Gln Ala Asp Ser
   385                 390                 395                 400
   ggt atc cgc ctg cac gct ctg cgt gtg gac ggc ggc gca gtg gcc aac     1248
   Gly Ile Arg Leu His Ala Leu Arg Val Asp Gly Gly Ala Val Ala Asn
                   405                 410                 415
   aac ttc ctg atg cag ttc cag tca gat att ctc ggc acg cgg gtg gaa     1296
   Asn Phe Leu Met Gln Phe Gln Ser Asp Ile Leu Gly Thr Arg Val Glu
               420                 425                 430
   cgc ccg gaa gtg cgc gaa gtg acc gcg ctg ggc gcc gcc tat ctg gct     1344
   Arg Pro Glu Val Arg Glu Val Thr Ala Leu Gly Ala Ala Tyr Leu Ala
           435                 440                 445
   ggc ctg gcc gtc ggt tac tgg cag aac ctg gac gag ctg cag gaa aaa     1392
   Gly Leu Ala Val Gly Tyr Trp Gln Asn Leu Asp Glu Leu Gln Glu Lys
       450                 455                 460
   gcc gtc att gag cga gaa ttc cgc ccc ggc att gaa acc acc gag cgt      1440
   Ala Val Ile Glu Arg Glu Phe Arg Pro Gly Ile Glu Thr Thr Glu Arg
   465                 470                 475                 480
   aac tac cgt tac agc ggc tgg aag aaa gcg gtt aaa cgc gca atg gcg      1488
   Asn Tyr Arg Tyr Ser Gly Trp Lys Lys Ala Val Lys Arg Ala Met Ala
                   485                 490                 495
   tgg gaa gag cac gac aag taa                                          1509
   Trp Glu Glu His Asp Lys
               500
<210> 4
   <211> 502
   <212> PRT
   <213> Salmonella typhimurium
<400> 4
   Met Thr Glu Lys Lys Tyr Ile Val Ala Leu Asp Gln Gly Thr Thr Ser
   1               5                   10                  15
   Ser Arg Ala Val Val Met Asp His Asp Ala Asn Ile Val Ser Val Ser
               20                  25                  30
   Gln Arg Glu Phe Glu Gln Ile Tyr Pro Lys Pro Gly Trp Val Glu His
           35                  40                  45
   Asp Pro Met Glu Ile Trp Ala Ser Gln Ser Ser Thr Leu Val Glu Val
       50                  55                  60
   Leu Ala Lys Ala Asp Ile Ser Ser Asp Gln Ile Ala Ala Ile Gly Ile
   65                  70                  75                  80
   Thr Asn Gln Arg Glu Thr Ala Ile Val Trp Glu Arg Glu Thr Gly Lys
                   85                  90                  95
   Pro Ile Tyr Asn Ala Ile Val Trp Gln Cys Arg Arg Thr Ala Asp Ile
               100                 105                 110
   Cys Glu Gln Leu Lys Arg Asp Gly Leu Glu Asp Tyr Ile Arg Asp Asn
           115                 120                 125
   Thr Gly Leu Val Val Asp Pro Tyr Phe Ser Gly Thr Lys Val Lys Trp
       130                 135                 140
   Ile Leu Asp His Val Glu Gly Ser Arg Glu Arg Ala Lys Arg Gly Glu
   145                 150                 155                 160
   Leu Leu Phe Gly Thr Val Asp Thr Trp Leu Ile Trp Lys Met Thr Gln
                   165                 170                 175
   Gly Arg Val His Val Thr Asp Tyr Thr Asn Ala Ser Arg Thr Met Leu
               180                 185                 190
   Phe Asn Ile His Asp Leu Asp Trp Asp Asp Lys Met Leu Asp Val Leu
   195 200 205
   Asp Ile Pro Arg Ala Met Leu Pro Gln Val Arg Lys Ser Ser Glu Val
   210 215 220
   Tyr Gly Gln Thr Asn Ile Gly Gly Lys Gly Gly Thr Arg Ile Pro Ile
   225 230 235 240
   Ala Gly Ile Ala Gly Asp Gln Gln Ala Ala Leu Phe Gly Gln Leu Cys
   245 250 255
   Val Lys Glu Gly Met Ala Lys Asn Thr Tyr Gly Thr Gly Cys Phe Met
   260 265 270
   Leu Met Asn Thr Gly Glu Lys Ala Val Lys Ser Glu Asn Gly Leu Leu
   275 280 285
   Ile Thr Ile Ala Cys Gly Pro Ser Gly Glu Val Asn Tyr Ala Leu Glu
   290 295 300
   Gly Ala Val Phe Met Ala Gly Ala Ser Ile Gln Trp Leu Arg Asp Glu
   305 310 315 320
   Met Lys Leu Ile Ser Asp Ala Phe Asp Ser Glu Tyr Phe Ala Thr Lys
   325 330 335
   Val Lys Asp Thr Asn Gly Val Tyr Val Val Pro Ala Phe Thr Gly Leu
   340 345 350
   Gly Ala Pro Tyr Trp Asp Pro Tyr Ala Arg Gly Ala Ile Phe Gly Leu
   355 360 365
   Thr Arg Gly Val Asn Ser Asn His Ile Ile Arg Ala Thr Leu Glu Ser
   370 375 380
   Ile Ala Tyr Gln Thr Arg Asp Val Leu Glu Ala Met Gln Ala Asp Ser
   385 390 395 400
   Gly Ile Arg Leu His Ala Leu Arg Val Asp Gly Gly Ala Val Ala Asn
   405 410 415
   Asn Phe Leu Met Gln Phe Gln Ser Asp Ile Leu Gly Thr Arg Val Glu
   420 425 430
   Arg Pro Glu Val Arg Glu Val Thr Ala Leu Gly Ala Ala Tyr Leu Ala
   435 440 445
   Gly Leu Ala Val Gly Tyr Trp Gln Asn Leu Asp Glu Leu Gln Glu Lys
   450 455 460
   Ala Val Ile Glu Arg Glu Phe Arg Pro Gly Ile Glu Thr Thr Glu Arg
   465                 470                 475                 480
   Asn Tyr Arg Tyr Ser Gly Trp Lys Lys Ala Val Lys Arg Ala Met Ala
                   485                 490                 495
   Trp Glu Glu His Asp Lys
               500
<210> 5
   <211> 1509
   <212> DNA
   <213> Shigella flexneri
<220>
   <221> CDS
   <222> (1)..(1509)
   <223> glpK Kodierregion
<400> 5
   atg act gaa aaa aaa tat atc gtt gcg ctc gac cag ggc acc acc agc      48
   Met Thr Glu Lys Lys Tyr Ile Val Ala Leu Asp Gln Gly Thr Thr Ser
   1               5                   10                  15
   tcc cgc gcg gtc gta atg gat cac gat gcc aat atc att agc gtg tcg      96
   Ser Arg Ala Val Val Met Asp His Asp Ala Asn Ile Ile Ser Val Ser
               20                  25                  30
   cag cgc gaa ttt gag caa atc tac cca aaa cca ggt tgg gta gaa cac     144
   Gln Arg Glu Phe Glu Gln Ile Tyr Pro Lys Pro Gly Trp Val Glu His
           35                  40                  45
   gac cca atg gaa atc tgg gcc acc caa agc tcc acg ctg gta gaa gtg     192
   Asp Pro Met Glu Ile Trp Ala Thr Gln Ser Ser Thr Leu Val Glu Val
       50                  55                  60
   ctg gcg aaa gcc gat atc agt tcc gat caa att gca gct atc ggt att     240
   Leu Ala Lys Ala Asp Ile Ser Ser Asp Gln Ile Ala Ala Ile Gly Ile
   65                  70                  75                  80
   acg aac cag cgt gaa acc act att gtc tgg gaa aaa gaa acc ggc aag     288
   Thr Asn Gln Arg Glu Thr Thr Ile Val Trp Glu Lys Glu Thr Gly Lys
                   85                  90                  95
   cct atc tat aac gcc att gtc tgg cag tgc cgt cgt acc gca gaa atc     336
   Pro Ile Tyr Asn Ala Ile Val Trp Gln Cys Arg Arg Thr Ala Glu Ile
               100                 105                 110
   tgc gag cat tta aaa cgt gac ggt tta gaa gat tat atc cgc agc aat     384
   Cys Glu His Leu Lys Arg Asp Gly Leu Glu Asp Tyr Ile Arg Ser Asn
           115                 120                 125
   acc ggt ctg gtg att gac ccg tac ttt tct ggc acc aaa gtg aag tgg     432
   Thr Gly Leu Val Ile Asp Pro Tyr Phe Ser Gly Thr Lys Val Lys Trp
       130                 135                 140
   atc ctc gac cat gtg gaa ggc tct cac gag cgt gca cgt cgt ggt gaa     480
   Ile Leu Asp His Val Glu Gly Ser His Glu Arg Ala Arg Arg Gly Glu
   145                 150                 155                 160
   ttg ctg ttt ggt acg gtt gat acg tgg ctt atc tgg aaa atg act cag     528
   Leu Leu Phe Gly Thr Val Asp Thr Trp Leu Ile Trp Lys Met Thr Gln
                   165                 170                 175
   ggc cgt gtc cat gtg acc gat tac acc aac gcc tct cgt acc atg ttg      576
   Gly Arg Val His Val Thr Asp Tyr Thr Asn Ala Ser Arg Thr Met Leu
               180                 185                 190
   ttc aac atc cat acc ctg gac tgg gac gac aaa atg ctg gaa gtg ctg      624
   Phe Asn Ile His Thr Leu Asp Trp Asp Asp Lys Met Leu Glu Val Leu
           195                 200                 205
   gat att ccg cgc gag atg ctg cca gaa gtg cgt cgt tct tcc gaa gta      672
   Asp Ile Pro Arg Glu Met Leu Pro Glu Val Arg Arg Ser Ser Glu Val
       210                 215                 220
   tac ggt cag act aac att ggc ggc aaa ggc ggc acg cgt att cca atc      720
   Tyr Gly Gln Thr Asn Ile Gly Gly Lys Gly Gly Thr Arg Ile Pro Ile
   225                 230                 235                 240
   tcc ggg atc gcc ggt gac cag cag gcc gcg ctg ttt ggt cag ttg tgc      768
   Ser Gly Ile Ala Gly Asp Gln Gln Ala Ala Leu Phe Gly Gln Leu Cys
                   245                 250                 255
   gtg aaa gaa ggg atg gcg aag aac acc tat ggc act ggc tgc ttt atg      816
   Val Lys Glu Gly Met Ala Lys Asn Thr Tyr Gly Thr Gly Cys Phe Met
               260                 265                 270
   ctg atg aac act ggc gag aaa gcg gtg aaa tca gaa aac ggc ctg ctg      864
   Leu Met Asn Thr Gly Glu Lys Ala Val Lys Ser Glu Asn Gly Leu Leu
           275                 280                 285
   acc acc atc gcc tgc ggc ccg act ggc gaa gtg aac tat gcg ttg gaa      912
   Thr Thr Ile Ala Cys Gly Pro Thr Gly Glu Val Asn Tyr Ala Leu Glu
       290                 295                 300
   ggt gcg gtg ttt atg gca ggc gca tcc att cag tgg ctg cgc gat gag      960
   Gly Ala Val Phe Met Ala Gly Ala Ser Ile Gln Trp Leu Arg Asp Glu
   305                 310                 315                 320
   atg aag ttg att aac gac gcc tac gat tcc gaa tat ttc gcc acc aaa     1008
   Met Lys Leu Ile Asn Asp Ala Tyr Asp Ser Glu Tyr Phe Ala Thr Lys
                   325                 330                 335
   gtg caa aac acc aat ggt gtg tat gtg gtt ccg gca ttt acc ggg ctg     1056
   Val Gln Asn Thr Asn Gly Val Tyr Val Val Pro Ala Phe Thr Gly Leu
               340                 345                 350
   ggt gcg ccg tac tgg gac ccg tat gcg cgc ggg gcg att ttc ggt ctg     1104
   Gly Ala Pro Tyr Trp Asp Pro Tyr Ala Arg Gly Ala Ile Phe Gly Leu
           355                 360                 365
   act cgt ggg gtg aac gct aac cac att ata cgc gcg acg ctg gag tct     1152
   Thr Arg Gly Val Asn Ala Asn His Ile Ile Arg Ala Thr Leu Glu Ser
       370                 375                 380
   att gct tat cag acg cgt gac gtg ctg gaa gcg atg cag gcc gac tct     1200
   Ile Ala Tyr Gln Thr Arg Asp Val Leu Glu Ala Met Gln Ala Asp Ser
   385                 390                 395                 400
   ggt atc cgt ctg cac gcc ctg cgc gtg gat ggt ggc gca gta gca aac     1248
   Gly Ile Arg Leu His Ala Leu Arg Val Asp Gly Gly Ala Val Ala Asn
                   405                 410                 415
   aat ttc ctg atg cag ttc cag tcc gat att ctc ggc acc cgc gtt gag     1296
   Asn Phe Leu Met Gln Phe Gln Ser Asp Ile Leu Gly Thr Arg Val Glu
               420                 425                 430
   cgc ccg gaa gtg cgc gaa gtc acc gca ttg ggt gcg gcc tat ctc gca     1344
   Arg Pro Glu Val Arg Glu Val Thr Ala Leu Gly Ala Ala Tyr Leu Ala
           435                 440                 445
   ggc ctg gcg gtt ggc ttc tgg cag aac ctc gac gag ctg caa gag aaa     1392
   Gly Leu Ala Val Gly Phe Trp Gln Asn Leu Asp Glu Leu Gln Glu Lys
       450                 455                 460
   gcg gtg att gag cgc gag ttc cgt cca ggc atc gaa acc act gag cgt      1440
   Ala Val Ile Glu Arg Glu Phe Arg Pro Gly Ile Glu Thr Thr Glu Arg
   465                 470                 475                 480
   aat tac cgt tac gca ggc tgg aaa aaa gcg gtc aaa cgc gcg atg gcg      1488
   Asn Tyr Arg Tyr Ala Gly Trp Lys Lys Ala Val Lys Arg Ala Met Ala
                   485                 490                 495
   tgg gaa gaa cac gac gag taa                                          1509
   Trp Glu Glu His Asp Glu
               500
<210> 6
   <211> 502
   <212> PRT
   <213> Shigella flexneri
<400> 6
   Met Thr Glu Lys Lys Tyr Ile Val Ala Leu Asp Gln Gly Thr Thr Ser
   1               5                   10                  15
   Ser Arg Ala Val Val Met Asp His Asp Ala Asn Ile Ile Ser Val Ser
               20                  25                  30
   Gln Arg Glu Phe Glu Gln Ile Tyr Pro Lys Pro Gly Trp Val Glu His
           35                  40                  45
   Asp Pro Met Glu Ile Trp Ala Thr Gln Ser Ser Thr Leu Val Glu Val
       50                  55                  60
   Leu Ala Lys Ala Asp Ile Ser Ser Asp Gln Ile Ala Ala Ile Gly Ile
   65                  70                  75                  80
   Thr Asn Gln Arg Glu Thr Thr Ile Val Trp Glu Lys Glu Thr Gly Lys
                   85                  90                  95
   Pro Ile Tyr Asn Ala Ile Val Trp Gln Cys Arg Arg Thr Ala Glu Ile
               100                 105                 110
   Cys Glu His Leu Lys Arg Asp Gly Leu Glu Asp Tyr Ile Arg Ser Asn
           115                 120                 125
   Thr Gly Leu Val Ile Asp Pro Tyr Phe Ser Gly Thr Lys Val Lys Trp
       130                 135                 140
   Ile Leu Asp His Val Glu Gly Ser His Glu Arg Ala Arg Arg Gly Glu
   145                 150                 155                 160
   Leu Leu Phe Gly Thr Val Asp Thr Trp Leu Ile Trp Lys Met Thr Gln
                   165                 170                 175
   Gly Arg Val His Val Thr Asp Tyr Thr Asn Ala Ser Arg Thr Met Leu
               180                 185                 190
   Phe Asn Ile His Thr Leu Asp Trp Asp Asp Lys Met Leu Glu Val Leu
   195 200 205
   Asp Ile Pro Arg Glu Met Leu Pro Glu Val Arg Arg Ser Ser Glu Val
   210 215 220
   Tyr Gly Gln Thr Asn Ile Gly Gly Lys Gly Gly Thr Arg Ile Pro Ile
   225 230 235 240
   Ser Gly Ile Ala Gly Asp Gln Gln Ala Ala Leu Phe Gly Gln Leu Cys
   245 250 255
   Val Lys Glu Gly Met Ala Lys Asn Thr Tyr Gly Thr Gly Cys Phe Met
   260 265 270
   Leu Met Asn Thr Gly Glu Lys Ala Val Lys Ser Glu Asn Gly Leu Leu
   275 280 285
   Thr Thr Ile Ala Cys Gly Pro Thr Gly Glu Val Asn Tyr Ala Leu Glu
   290 295 300
   Gly Ala Val Phe Met Ala Gly Ala Ser Ile Gln Trp Leu Arg Asp Glu
   305 310 315 320
   Met Lys Leu Ile Asn Asp Ala Tyr Asp Ser Glu Tyr Phe Ala Thr Lys
   325 330 335
   Val Gln Asn Thr Asn Gly Val Tyr Val Val Pro Ala Phe Thr Gly Leu
   340 345 350
   Gly Ala Pro Tyr Trp Asp Pro Tyr Ala Arg Gly Ala Ile Phe Gly Leu
   355 360 365
   Thr Arg Gly Val Asn Ala Asn His Ile Ile Arg Ala Thr Leu Glu Ser
   370 375 380
   Ile Ala Tyr Gln Thr Arg Asp Val Leu Glu Ala Met Gln Ala Asp Ser
   385 390 395 400
   Gly Ile Arg Leu His Ala Leu Arg Val Asp Gly Gly Ala Val Ala Asn
   405 410 415
   Asn Phe Leu Met Gln Phe Gln Ser Asp Ile Leu Gly Thr Arg Val Glu
   420 425 430
   Arg Pro Glu Val Arg Glu Val Thr Ala Leu Gly Ala Ala Tyr Leu Ala
   435 440 445
   Gly Leu Ala Val Gly Phe Trp Gln Asn Leu Asp Glu Leu Gln Glu Lys
   450 455 460
   Ala Val Ile Glu Arg Glu Phe Arg Pro Gly Ile Glu Thr Thr Glu Arg
   465 470 475 480
   Asn Tyr Arg Tyr Ala Gly Trp Lys Lys Ala Val Lys Arg Ala Met Ala
   485 490 495
   Trp Glu Glu His Asp Glu
   500
<210> 7
   <211> 2150
   <212> DNA
   <213> Escherichia coli
<220>
   <221> CDS
   <222> (109)..(1617)
   <223> glpK Kodierregion
<400> 7
   tgattggtcg ccatttgcct tgcgatatct gtgttgtgga agaaaaggaa accacaactc      60
   cttcagaaca aaaagcttcg ctgtaatatg actacgggac aattaaac atg act gaa      117
                                                        Met Thr Glu
                                                        1
   aaa aaa tat atc gtt gcg ctc gac cag ggc acc acc agc tcc cgc gcg      165
   Lys Lys Tyr Ile Val Ala Leu Asp Gln Gly Thr Thr Ser Ser Arg Ala
       5                   10                  15
   gtc gta atg gat cac gat gcc aat atc att agc gtg tcg cag cgc gaa      213
   Val Val Met Asp His Asp Ala Asn Ile Ile Ser Val Ser Gln Arg Glu
   20                  25                  30                  35
   ttt gag caa atc tac cca aaa cca ggt tgg gta gaa cac gac cca atg      261
   Phe Glu Gln Ile Tyr Pro Lys Pro Gly Trp Val Glu His Asp Pro Met
                   40                  45                  50
   gaa atc tgg gcc acc caa agc tcc acg ctg gta gaa gtg ctg gcg aaa      309
   Glu Ile Trp Ala Thr Gln Ser Ser Thr Leu Val Glu Val Leu Ala Lys
               55                  60                  65
   gcc gat atc agt tcc gat caa att gca gct atc ggt att acg aac cag      357
   Ala Asp Ile Ser Ser Asp Gln Ile Ala Ala Ile Gly Ile Thr Asn Gln
           70                  75                  80
   cgt gaa acc act att gtc tgg gaa aaa gaa acc ggc aag cct atc tat      405
   Arg Glu Thr Thr Ile Val Trp Glu Lys Glu Thr Gly Lys Pro Ile Tyr
       85                  90                  95
   aac gcc att gtc tgg cag tgc cgt cgt acc gca gaa atc tgc gag cat      453
   Asn Ala Ile Val Trp Gln Cys Arg Arg Thr Ala Glu Ile Cys Glu His
   100                 105                 110                 115
   tta aaa cgt gac ggt tta gaa gat tat atc cgc agc aat acc ggt ctg      501
   Leu Lys Arg Asp Gly Leu Glu Asp Tyr Ile Arg Ser Asn Thr Gly Leu
                   120                 125                 130
   gtg att gac ccg tac ttt tct ggc acc aaa gtg aag tgg atc ctc gac      549
   Val Ile Asp Pro Tyr Phe Ser Gly Thr Lys Val Lys Trp Ile Leu Asp
               135                 140                 145
   cat gtg gaa ggc tct cgc gag cgt gca cgt cgt ggt gaa ttg ctg ttt      597
   His Val Glu Gly Ser Arg Glu Arg Ala Arg Arg Gly Glu Leu Leu Phe
               150                       155                       160
   ggt acg gtt gat acg tgg ctt atc tgg aaa atg act cag ggc cgt gtc        645
   Gly Thr Val Asp Thr Trp Leu Ile Trp Lys Met Thr Gln Gly Arg Val
       165                     170                     175
   cat gtg acc gat tac acc aac gcc tct cgt acc atg ttg ttc aac atc        693
   His Val Thr Asp Tyr Thr Asn Ala Ser Arg Thr Met Leu Phe Asn Ile
   180                     185                     190                 195
   cat acc ctg gac tgg gac gac aaa atg ctg gaa gtg ctg gat att ccg        741
   His Thr Leu Asp Trp Asp Asp Lys Met Leu Glu Val Leu Asp Ile Pro
                   200                     205                     210
   cgc gag atg ctg cca gaa gtg cgt cgt tct tcc gaa gta tac ggt cag        789
   Arg Glu Met Leu Pro Glu Val Arg Arg Ser Ser Glu Val Tyr Gly Gln
               215                     220                     225
   act aac att ggc ggc aaa ggc ggc acg cgt att cca atc tcc ggg atc        837
   Thr Asn Ile Gly Gly Lys Gly Gly Thr Arg Ile Pro Ile Ser Gly Ile
           230                     235                     240
   gcc ggt gac cag cag gcc gcg ctg ttt ggt cag ttg tgc gtg aaa gaa        885
   Ala Gly Asp Gln Gln Ala Ala Leu Phe Gly Gln Leu Cys Val Lys Glu
       245                     250                     255
   ggg atg gcg aag aac acc tat ggc act ggc tgc ttt atg ctg atg aac        933
   Gly Met Ala Lys Asn Thr Tyr Gly Thr Gly Cys Phe Met Leu Met Asn
   260                     265                     270                 275
   act ggc gag aaa gcg gtg aaa tca gaa aac ggc ctg ctg acc acc atc        981
   Thr Gly Glu Lys Ala Val Lys Ser Glu Asn Gly Leu Leu Thr Thr Ile
                   280                     285                     290
   gcc tgc ggc ccg act ggc gaa gtg aac tat gcg ttg gaa ggt gcg gtg       1029
   Ala Cys Gly Pro Thr Gly Glu Val Asn Tyr Ala Leu Glu Gly Ala Val
               295                     300                     305
   ttt atg gca ggc gca tcc att cag tgg ctg cgc gat gaa atg aag ttg       1077
   Phe Met Ala Gly Ala Ser Ile Gln Trp Leu Arg Asp Glu Met Lys Leu
           310                     315                     320
   att aac gac gcc tac gat tcc gaa tat ttc gcc acc aaa gtg caa aac       1125
   Ile Asn Asp Ala Tyr Asp Ser Glu Tyr Phe Ala Thr Lys Val Gln Asn
       325                     330                     335
   acc aat ggt gtg tat gtg gtt ccg gca ttt acc ggg ctg ggt gcg ccg       1173
   Thr Asn Gly Val Tyr Val Val Pro Ala Phe Thr Gly Leu Gly Ala Pro
   340                     345                     350                 355
   tac tgg gac ccg tat gcg cgc ggg gcg att ttc ggt ctg act cgt ggg       1221
   Tyr Trp Asp Pro Tyr Ala Arg Gly Ala Ile Phe Gly Leu Thr Arg Gly
                   360                     365                     370
   gtg aac gct aac cac att ata cgc gcg acg ctg gag tct att gct tat       1269
   Val Asn Ala Asn His Ile Ile Arg Ala Thr Leu Glu Ser Ile Ala Tyr
               375                     380                     385
   cag acg cgt gac gtg ctg gaa gcg atg cag gcc gac tct ggt atc cgt       1317
   Gln Thr Arg Asp Val Leu Glu Ala Met Gln Ala Asp Ser Gly Ile Arg
           390                     395                     400
   ctg cac gcc ctg cgc gtg gat ggt ggc gca gta gca aac aat ttc ctg       1365
   Leu His Ala Leu Arg Val Asp Gly Gly Ala Val Ala Asn Asn Phe Leu
       405                     410                     415
   atg cag ttc cag tcc gat att ctc ggc acc cgc gtt gag cgc ccg gaa       1413
   Met Gln Phe Gln Ser Asp Ile Leu Gly Thr Arg Val Glu Arg Pro Glu
   420                     425                     430                 435
   gtg cgc gaa gtc acc gca ttg ggt gcg gcc tat ctc gca ggc ctg gcg      1461
   Val Arg Glu Val Thr Ala Leu Gly Ala Ala Tyr Leu Ala Gly Leu Ala
                   440                 445                 450
   gtt ggc ttc tgg cag aac ctc gac gag ctg caa gag aaa gcg gtg att      1509
   Val Gly Phe Trp Gln Asn Leu Asp Glu Leu Gln Glu Lys Ala Val Ile
               455                 460                 465
   gag cgc gag ttc cgt cca ggc atc gaa acc act gag cgt aat tac cgt      1557
   Glu Arg Glu Phe Arg Pro Gly Ile Glu Thr Thr Glu Arg Asn Tyr Arg
           470                 475                 480
   tac gca ggc tgg aaa aaa gcg gtt aaa cgc gcg atg gcg tgg gaa gaa      1605
   Tyr Ala Gly Trp Lys Lys Ala Val Lys Arg Ala Met Ala Trp Glu Glu
       485                 490                 495
   cac gac gaa taa tgtaaatgcc gaatgaagcg tttatgccgc atccggtagt          1657
   His Asp Glu
   500
   cccgaaacgt gcgggggcaa ccccgcacac atcaataatc cctcccttcc cctgtgctac    1717
   acttcgcgcc attccttact gcttagagtt tgctatgaga cgagaacttg ccatcgaatt    1777
   ttcccgcgtc accgaatcag cggcgctggc tggctacaaa tggttaggac gcggcgataa    1837
   aaacaccgcg gacggcgcgg cggtaaacgc catgcgtatt atgctcaacc aggtcaacat    1897
   tgacggcacc atcgtcattg gtgaaggtga aatcgacgaa gcaccgatgc tctacattgg    1957
   tgaaaaagtc ggtactggtc gcggcgacgc ggtagatatt gctgttgatc cgattgaagg    2017
   cacgcgcatg acggcgatgg gccaggctaa cgcgctggcg gtgctggcag taggcgataa    2077
   aggctgcttc ctcaatgcgc cggatatgta tatggagaag ctgattgtcg ggccgggagc    2137
   caaaggcacc att                                                       2150
<210> 8
   <211> 502
   <212> PRT
   <213> Escherichia coli
<400> 8
   Met Thr Glu Lys Lys Tyr Ile Val Ala Leu Asp Gln Gly Thr Thr Ser
   1               5                   10                  15
   Ser Arg Ala Val Val Met Asp His Asp Ala Asn Ile Ile Ser Val Ser
               20                  25                  30
   Gln Arg Glu Phe Glu Gln Ile Tyr Pro Lys Pro Gly Trp Val Glu His
           35                  40                  45
   Asp Pro Met Glu Ile Trp Ala Thr Gln Ser Ser Thr Leu Val Glu Val
       50                  55                  60
   Leu Ala Lys Ala Asp Ile Ser Ser Asp Gln Ile Ala Ala Ile Gly Ile
   65                  70                  75                  80
   Thr Asn Gln Arg Glu Thr Thr Ile Val Trp Glu Lys Glu Thr Gly Lys
                   85                  90                  95
   Pro Ile Tyr Asn Ala Ile Val Trp Gln Cys Arg Arg Thr Ala Glu Ile
               100                 105                 110
   Cys Glu His Leu Lys Arg Asp Gly Leu Glu Asp Tyr Ile Arg Ser Asn
           115                 120                 125
   Thr Gly Leu Val Ile Asp Pro Tyr Phe Ser Gly Thr Lys Val Lys Trp
       130                 135                 140
   Ile Leu Asp His Val Glu Gly Ser Arg Glu Arg Ala Arg Arg Gly Glu
   145                 150                 155                 160
   Leu Leu Phe Gly Thr Val Asp Thr Trp Leu Ile Trp Lys Met Thr Gln
                   165                 170                 175
   Gly Arg Val His Val Thr Asp Tyr Thr Asn Ala Ser Arg Thr Met Leu
               180                 185                 190
   Phe Asn Ile His Thr Leu Asp Trp Asp Asp Lys Met Leu Glu Val Leu
           195                 200                 205
   Asp Ile Pro Arg Glu Met Leu Pro Glu Val Arg Arg Ser Ser Glu Val
       210                 215                 220
   Tyr Gly Gln Thr Asn Ile Gly Gly Lys Gly Gly Thr Arg Ile Pro Ile
   225                 230                 235                 240
   Ser Gly Ile Ala Gly Asp Gln Gln Ala Ala Leu Phe Gly Gln Leu Cys
                   245                 250                 255
   Val Lys Glu Gly Met Ala Lys Asn Thr Tyr Gly Thr Gly Cys Phe Met
               260                 265                 270
   Leu Met Asn Thr Gly Glu Lys Ala Val Lys Ser Glu Asn Gly Leu Leu
           275                 280                 285
   Thr Thr Ile Ala Cys Gly Pro Thr Gly Glu Val Asn Tyr Ala Leu Glu
       290                 295                 300
   Gly Ala Val Phe Met Ala Gly Ala Ser Ile Gln Trp Leu Arg Asp Glu
   305                 310                 315                 320
   Met Lys Leu Ile Asn Asp Ala Tyr Asp Ser Glu Tyr Phe Ala Thr Lys
                   325                 330                 335
   Val Gln Asn Thr Asn Gly Val Tyr Val Val Pro Ala Phe Thr Gly Leu
               340                 345                 350
   Gly Ala Pro Tyr Trp Asp Pro Tyr Ala Arg Gly Ala Ile Phe Gly Leu
           355                 360                 365
   Thr Arg Gly Val Asn Ala Asn His Ile Ile Arg Ala Thr Leu Glu Ser
       370                 375                 380
   Ile Ala Tyr Gln Thr Arg Asp Val Leu Glu Ala Met Gln Ala Asp Ser
   385                 390                 395                 400
   Gly Ile Arg Leu His Ala Leu Arg Val Asp Gly Gly Ala Val Ala Asn
                   405                 410                 415
   Asn Phe Leu Met Gln Phe Gln Ser Asp Ile Leu Gly Thr Arg Val Glu
               420                 425                 430
   Arg Pro Glu Val Arg Glu Val Thr Ala Leu Gly Ala Ala Tyr Leu Ala
           435                 440                 445
   Gly Leu Ala Val Gly Phe Trp Gln Asn Leu Asp Glu Leu Gln Glu Lys
       450                 455                 460
   Ala Val Ile Glu Arg Glu Phe Arg Pro Gly Ile Glu Thr Thr Glu Arg
   465                 470                 475                 480
   Asn Tyr Arg Tyr Ala Gly Trp Lys Lys Ala Val Lys Arg Ala Met Ala
                   485                 490                 495
   Trp Glu Glu His Asp Glu
               500
<210> 9
   <211> 20
   <212> DNA
   <213> Escherichia coli
<220>
   <221> primer
   <222> (1)..(20)
   <223> glpK_3
<400> 9
   cgattacacc aacgcctctc 20
<210> 10
   <211> 40
   <212> DNA
   <213> artificial sequence
<220>
   <223> "gene SOEing"-primer
<220>
   <221> primer
   <222> (1)..(40)
   <223> glpK_4_SOEing
<400> 10
   tccggacgct caacgcgggt atcgagaata tcggactgga 40
<210> 11
   <211> 40
   <212> DNA
   <213> artificial sequence
<220>
   <223> "gene SOEing"-primer
<220>
   <221> primer
   <222> (1)..(40)
   <223> glpK_5_SOEing
<400> 11
   tctcgatacc cgcgttgagc gtccggaagt gcgcgaagtc 40
<210> 12
   <211> 20
   <212> DNA
   <213> Escherichia coli
<220>
   <221> primer
   <222> (1)..(20)
   <223> glpK_6
<400> 12
   gcccgacaat cagcttctcc 20
<210> 13
   <211> 1477
   <212> DNA
   <213> Escherichia coli
<220>
   <221> mutation
   <222> (738)..(739)
   <223> transitions gc -> at
<400> 13
   cgattacacc aacgcctctc gtaccatgtt gttcaacatc cataccctgg actgggacga 60
   caaaatgctg gaagtgctgg atattccgcg cgagatgctg ccagaagtgc gtcgttcttc 120
   cgaagtatac ggtcagacta acattggcgg caaaggcggc acgcgtattc caatctccgg 180
   gatcgccggt gaccagcagg ccgcgctgtt tggtcagttg tgcgtgaaag aagggatggc 240
   gaagaacacc tatggcactg gctgctttat gctgatgaac actggcgaga aagcggtgaa 300
   atcagaaaac ggcctgctga ccaccatcgc ctgcggcccg actggcgaag tgaactatgc 360
   gttggaaggt gcggtgttta tggcaggcgc atccattcag tggctgcgcg atgaaatgaa 420
   gttgattaac gacgcctacg attccgaata tttcgccacc aaagtgcaaa acaccaatgg 480
   tgtgtatgtg gttccggcat ttaccgggct gggtgcgccg tactgggacc cgtatgcgcg 540
   cggggcgatt ttcggtctga ctcgtggggt gaacgctaac cacattatac gcgcgacgct 600
   ggagtctatt gcttatcaga cgcgtgacgt gctggaagcg atgcaggccg actctggtat 660
   ccgtctgcac gccctgcgcg tggatggtgg cgcagtagca aacaatttcc tgatgcagtt 720
   ccagtccgat attctcgata cccgcgttga gcgtccggaa gtgcgcgaag tcaccgcatt 780
   gggtgcggcc tatctcgcag gcctggcggt tggcttctgg cagaacctcg acgagctgca 840
   agagaaagcg gtgattgagc gcgagttccg tccaggcatc gaaaccactg agcgtaatta 900
   ccgttacgca ggctggaaaa aagcggttaa acgcgcgatg gcgtgggaag aacacgacga 960
   ataatgtaaa tgccgaatga agcgtttatg ccgcatccgg tagtcccgaa acgtgcgggg 1020
   gcaaccccgc acacatcaat aatccctccc ttcccctgtg ctacacttcg cgccattcct 1080
   tactgcttag agtttgctat gagacgagaa cttgccatcg aattttcccg cgtcaccgaa 1140
   tcagcggcgc tggctggcta caaatggtta ggacgcggcg ataaaaacac cgcggacggc 1200
   gcggcggtaa acgccatgcg tattatgctc aaccaggtca acattgacgg caccatcgtc 1260
   attggtgaag gtgaaatcga cgaagcaccg atgctctaca ttggtgaaaa agtcggtact 1320
   ggtcgcggcg acgcggtaga tattgctgtt gatccgattg aaggcacgcg catgacggcg 1380
   atgggccagg ctaacgcgct ggcggtgctg gcagtaggcg ataaaggctg cttcctcaat 1440
   gcgccggata tgtatatgga gaagctgatt gtcgggc 1477
<210> 14
   <211> 29
   <212> DNA
   <213> artificial sequence
<220>
   <223> primer with SalI recognition site
<220>
   <221> primer
   <222> (1)..(29)
   <223> glpK_1
<400> 14
   cggtcgacat gactacggga caattaaac 29
<210> 15
   <211> 40
   <212> DNA
   <213> artificial sequence
<220>
   <223> "gene SOEing"-primer
<220>
   <221> primer
   <222> (1)..(40)
   <223> glpK-7_SOEing
<400> 15
   gaattcgcgt gccgcctttg ccatcaatgt tagtctgacc 40
<210> 16
   <211> 40
   <212> DNA
   <213> artificial sequence
<220>
   <223> "gene SOEing"-primer
<220>
   <221> primer
   <222> (1)..(40)
   <223> glpK_8_SOEing
<400> 16
   cattgatggc aaaggcggca cgcgaattcc aatctccggg 40
<210> 17
   <211> 28
   <212> DNA
   <213> artificial sequence
<220>
   <223> primer with SalI recognition site
<220>
   <221> primer
   <222> (1)..(28)
   <223> glpFK_2
<400> 17
   cggtcgactt attcgtcgtg ttcttccc 28
<210> 18
   <211> 1546
   <212> DNA
   <213> Escherichia coli
<220>
   <221> CDS
   <222> (30)..(1538)
   <223> glpK Kodierregion
<220>
   <221> mutation
   <222> (721)..(722)
   <223> transitions gc -> at
<400> 18
   cggtcgacat gactacggga caattaaac atg act gaa aaa aaa tat atc gtt        53
                                   Met Thr Glu Lys Lys Tyr Ile Val
                                   1               5
   gcg ctc gac cag ggc acc acc agc tcc cgc gcg gtc gta atg gat cac       101
   Ala Leu Asp Gln Gly Thr Thr Ser Ser Arg Ala Val Val Met Asp His
       10                  15                  20
   gat gcc aat atc att agc gtg tcg cag cgc gaa ttt gag caa atc tac       149
   Asp Ala Asn Ile Ile Ser Val Ser Gln Arg Glu Phe Glu Gln Ile Tyr
   25                  30                  35                  40
   cca aaa cca ggt tgg gta gaa cac gac cca atg gaa atc tgg gcc acc       197
   Pro Lys Pro Gly Trp Val Glu His Asp Pro Met Glu Ile Trp Ala Thr
                   45                  50                  55
   caa agc tcc acg ctg gta gaa gtg ctg gcg aaa gcc gat atc agt tcc       245
   Gln Ser Ser Thr Leu Val Glu Val Leu Ala Lys Ala Asp Ile Ser Ser
               60                  65                  70
   gat caa att gca gct atc ggt att acg aac cag cgt gaa acc act att       293
   Asp Gln Ile Ala Ala Ile Gly Ile Thr Asn Gln Arg Glu Thr Thr Ile
           75                  80                  85
   gtc tgg gaa aaa gaa acc ggc aag cct atc tat aac gcc att gtc tgg      341
   Val Trp Glu Lys Glu Thr Gly Lys Pro Ile Tyr Asn Ala Ile Val Trp
       90                  95                  100
   cag tgc cgt cgt acc gca gaa atc tgc gag cat tta aaa cgt gac ggt      389
   Gln Cys Arg Arg Thr Ala Glu Ile Cys Glu His Leu Lys Arg Asp Gly
   105                 110                 115                 120
   tta gaa gat tat atc cgc agc aat acc ggt ctg gtg att gac ccg tac      437
   Leu Glu Asp Tyr Ile Arg Ser Asn Thr Gly Leu Val Ile Asp Pro Tyr
                   125                 130                 135
   ttt tct ggc acc aaa gtg aag tgg atc ctc gac cat gtg gaa ggc tct      485
   Phe Ser Gly Thr Lys Val Lys Trp Ile Leu Asp His Val Glu Gly Ser
               140                 145                 150
   cgc gag cgt gca cgt cgt ggt gaa ttg ctg ttt ggt acg gtt gat acg      533
   Arg Glu Arg Ala Arg Arg Gly Glu Leu Leu Phe Gly Thr Val Asp Thr
           155                 160                 165
   tgg ctt atc tgg aaa atg act cag ggc cgt gtc cat gtg acc gat tac      581
   Trp Leu Ile Trp Lys Met Thr Gln Gly Arg Val His Val Thr Asp Tyr
       170                 175                 180
   acc aac gcc tct cgt acc atg ttg ttc aac atc cat acc ctg gac tgg      629
   Thr Asn Ala Ser Arg Thr Met Leu Phe Asn Ile His Thr Leu Asp Trp
   185                 190                 195                 200
   gac gac aaa atg ctg gaa gtg ctg gat att ccg cgc gag atg ctg cca      677
   Asp Asp Lys Met Leu Glu Val Leu Asp Ile Pro Arg Glu Met Leu Pro
                   205                 210                 215
   gaa gtg cgt cgt tct tcc gaa gta tac ggt cag act aac att gat ggc      725
   Glu Val Arg Arg Ser Ser Glu Val Tyr Gly Gln Thr Asn Ile Asp Gly
               220                 225                 230
   aaa ggc ggc acg cga att cca atc tcc ggg atc gcc ggt gac cag cag      773
   Lys Gly Gly Thr Arg Ile Pro Ile Ser Gly Ile Ala Gly Asp Gln Gln
           235                 240                 245
   gcc gcg ctg ttt ggt cag ttg tgc gtg aaa gaa ggg atg gcg aag aac      821
   Ala Ala Leu Phe Gly Gln Leu Cys Val Lys Glu Gly Met Ala Lys Asn
       250                 255                 260
   acc tat ggc act ggc tgc ttt atg ctg atg aac act ggc gag aaa gcg      869
   Thr Tyr Gly Thr Gly Cys Phe Met Leu Met Asn Thr Gly Glu Lys Ala
   265                 270                 275                 280
   gtg aaa tca gaa aac ggc ctg ctg acc acc atc gcc tgc ggc ccg act      917
   Val Lys Ser Glu Asn Gly Leu Leu Thr Thr Ile Ala Cys Gly Pro Thr
                   285                 290                 295
   ggc gaa gtg aac tat gcg ttg gaa ggt gcg gtg ttt atg gca ggc gca      965
   Gly Glu Val Asn Tyr Ala Leu Glu Gly Ala Val Phe Met Ala Gly Ala
               300                 305                 310
   tcc att cag tgg ctg cgc gat gaa atg aag ttg att aac gac gcc tac     1013
   Ser Ile Gln Trp Leu Arg Asp Glu Met Lys Leu Ile Asn Asp Ala Tyr
           315                 320                 325
   gat tcc gaa tat ttc gcc acc aaa gtg caa aac acc aat ggt gtg tat     1061
   Asp Ser Glu Tyr Phe Ala Thr Lys Val Gln Asn Thr Asn Gly Val Tyr
       330                 335                 340
   gtg gtt ccg gca ttt acc ggg ctg ggt gcg ccg tac tgg gac ccg tat     1109
   Val Val Pro Ala Phe Thr Gly Leu Gly Ala Pro Tyr Trp Asp Pro Tyr
   345                 350                 355                 360
   gcg cgc ggg gcg att ttc ggt ctg act cgt ggg gtg aac gct aac cac      1157
   Ala Arg Gly Ala Ile Phe Gly Leu Thr Arg Gly Val Asn Ala Asn His
                   365                 370                 375
   att ata cgc gcg acg ctg gag tct att gct tat cag acg cgt gac gtg      1205
   Ile Ile Arg Ala Thr Leu Glu Ser Ile Ala Tyr Gln Thr Arg Asp Val
               380                 385                 390
   ctg gaa gcg atg cag gcc gac tct ggt atc cgt ctg cac gcc ctg cgc      1253
   Leu Glu Ala Met Gln Ala Asp Ser Gly Ile Arg Leu His Ala Leu Arg
           395                 400                 405
   gtg gat ggt ggc gca gta gca aac aat ttc ctg atg cag ttc cag tcc      1301
   Val Asp Gly Gly Ala Val Ala Asn Asn Phe Leu Met Gln Phe Gln Ser
       410                 415                 420
   gat att ctc ggc acc cgc gtt gag cgc ccg gaa gtg cgc gaa gtc acc      1349
   Asp Ile Leu Gly Thr Arg Val Glu Arg Pro Glu Val Arg Glu Val Thr
   425                 430                 435                 440
   gca ttg ggt gcg gcc tat ctc gca ggc ctg gcg gtt ggc ttc tgg cag      1397
   Ala Leu Gly Ala Ala Tyr Leu Ala Gly Leu Ala Val Gly Phe Trp Gln
                   445                 450                 455
   aac ctc gac gag ctg caa gag aaa gcg gtg att gag cgc gag ttc cgt      1445
   Asn Leu Asp Glu Leu Gln Glu Lys Ala Val Ile Glu Arg Glu Phe Arg
               460                 465                 470
   cca ggc atc gaa acc act gag cgt aat tac cgt tac gca ggc tgg aaa      1493
   Pro Gly Ile Glu Thr Thr Glu Arg Asn Tyr Arg Tyr Ala Gly Trp Lys
           475                 480                 485
   aaa gcg gtt aaa cgc gcg atg gcg tgg gaa gaa cac gac gaa taa          1538
   Lys Ala Val Lys Arg Ala Met Ala Trp Glu Glu His Asp Glu
       490                 495                 500
   gtcgaccg                                                             1546
<210> 19
   <211> 502
   <212> PRT
   <213> Escherichia coli
<400> 19
   Met Thr Glu Lys Lys Tyr Ile Val Ala Leu Asp Gln Gly Thr Thr Ser
   1               5                   10                  15
   Ser Arg Ala Val Val Met Asp His Asp Ala Asn Ile Ile Ser Val Ser
               20                  25                  30
   Gln Arg Glu Phe Glu Gln Ile Tyr Pro Lys Pro Gly Trp Val Glu His
           35                  40                  45
   Asp Pro Met Glu Ile Trp Ala Thr Gln Ser Ser Thr Leu Val Glu Val
       50                  55                  60
   Leu Ala Lys Ala Asp Ile Ser Ser Asp Gln Ile Ala Ala Ile Gly Ile
   65                  70                  75                  80
   Thr Asn Gln Arg Glu Thr Thr Ile Val Trp Glu Lys Glu Thr Gly Lys
   85 90 95
   Pro Ile Tyr Asn Ala Ile Val Trp Gln Cys Arg Arg Thr Ala Glu Ile
   100 105 110
   Cys Glu His Leu Lys Arg Asp Gly Leu Glu Asp Tyr Ile Arg Ser Asn
   115 120 125
   Thr Gly Leu Val Ile Asp Pro Tyr Phe Ser Gly Thr Lys Val Lys Trp
   130 135 140
   Ile Leu Asp His Val Glu Gly Ser Arg Glu Arg Ala Arg Arg Gly Glu
   145 150 155 160
   Leu Leu Phe Gly Thr Val Asp Thr Trp Leu Ile Trp Lys Met Thr Gln
   165 170 175
   Gly Arg Val His Val Thr Asp Tyr Thr Asn Ala Ser Arg Thr Met Leu
   180 185 190
   Phe Asn Ile His Thr Leu Asp Trp Asp Asp Lys Met Leu Glu Val Leu
   195 200 205
   Asp Ile Pro Arg Glu Met Leu Pro Glu Val Arg Arg Ser Ser Glu Val
   210 215 220
   Tyr Gly Gln Thr Asn Ile Asp Gly Lys Gly Gly Thr Arg Ile Pro Ile
   225 230 235 240
   Ser Gly Ile Ala Gly Asp Gln Gln Ala Ala Leu Phe Gly Gln Leu Cys
   245 250 255
   Val Lys Glu Gly Met Ala Lys Asn Thr Tyr Gly Thr Gly Cys Phe Met
   260 265 270
   Leu Met Asn Thr Gly Glu Lys Ala Val Lys Ser Glu Asn Gly Leu Leu
   275 280 285
   Thr Thr Ile Ala Cys Gly Pro Thr Gly Glu Val Asn Tyr Ala Leu Glu
   290 295 300
   Gly Ala Val Phe Met Ala Gly Ala Ser Ile Gln Trp Leu Arg Asp Glu
   305 310 315 320
   Met Lys Leu Ile Asn Asp Ala Tyr Asp Ser Glu Tyr Phe Ala Thr Lys
   325 330 335
   Val Gln Asn Thr Asn Gly Val Tyr Val Val Pro Ala Phe Thr Gly Leu
   340 345 350
   Gly Ala Pro Tyr Trp Asp Pro Tyr Ala Arg Gly Ala Ile Phe Gly Leu
   355 360 365
   Thr Arg Gly Val Asn Ala Asn His Ile Ile Arg Ala Thr Leu Glu Ser
   370 375 380
   Ile Ala Tyr Gln Thr Arg Asp Val Leu Glu Ala Met Gln Ala Asp Ser
   385 390 395 400
   Gly Ile Arg Leu His Ala Leu Arg Val Asp Gly Gly Ala Val Ala Asn
   405 410 415
   Asn Phe Leu Met Gln Phe Gln Ser Asp Ile Leu Gly Thr Arg Val Glu
   420 425 430
   Arg Pro Glu Val Arg Glu Val Thr Ala Leu Gly Ala Ala Tyr Leu Ala
   435 440 445
   Gly Leu Ala Val Gly Phe Trp Gln Asn Leu Asp Glu Leu Gln Glu Lys
   450 455 460
   Ala Val Ile Glu Arg Glu Phe Arg Pro Gly Ile Glu Thr Thr Glu Arg
   465 470 475 480
   Asn Tyr Arg Tyr Ala Gly Trp Lys Lys Ala Val Lys Arg Ala Met Ala
   485 490 495
   Trp Glu Glu His Asp Glu
   500
<210> 20
   <211> 502
   <212> PRT
   <213> Escherichia coli
<400> 20
   Met Thr Glu Lys Lys Tyr Ile Val Ala Leu Asp Gln Gly Thr Thr Ser
   1 5 10 15
   Ser Arg Ala Val Val Met Asp His Asp Ala Asn Ile Ile Ser Val Ser
   20 25 30
   Gln Arg Glu Phe Glu Gln Ile Tyr Pro Lys Pro Gly Trp Val Glu His
   35 40 45
   Asp Pro Met Glu Ile Trp Ala Thr Gln Ser Ser Thr Leu Val Glu Val
   50 55 60
   Leu Ala Lys Ala Asp Ile Ser Ser Asp Gln Ile Ala Ala Ile Gly Ile
   65 70 75 80
   Thr Asn Gln Arg Glu Thr Thr Ile Val Trp Glu Lys Glu Thr Gly Lys
   85 90 95
   Pro Ile Tyr Asn Ala Ile Val Trp Gln Cys Arg Arg Thr Ala Glu Ile
               100                 105                 110
   Cys Glu His Leu Lys Arg Asp Gly Leu Glu Asp Tyr Ile Arg Ser Asn
           115                 120                 125
   Thr Gly Leu Val Ile Asp Pro Tyr Phe Ser Gly Thr Lys Val Lys Trp
       130                 135                 140
   Ile Leu Asp His Val Glu Gly Ser Arg Glu Arg Ala Arg Arg Gly Glu
   145                 150                 155                 160
   Leu Leu Phe Gly Thr Val Asp Thr Trp Leu Ile Trp Lys Met Thr Gln
                   165                 170                 175
   Gly Arg Val His Val Thr Asp Tyr Thr Asn Ala Ser Arg Thr Met Leu
               180                 185                 190
   Phe Asn Ile His Thr Leu Asp Trp Asp Asp Lys Met Leu Glu Val Leu
           195                 200                 205
   Asp Ile Pro Arg Glu Met Leu Pro Glu Val Arg Arg Ser Ser Glu Val
       210                 215                 220
   Tyr Gly Gln Thr Asn Ile Gly Gly Lys Gly Gly Thr Ser Ile Pro Ile
   225                 230                 235                 240
   Ser Gly Ile Ala Gly Asp Gln Gln Ala Ala Leu Phe Gly Gln Leu Cys
                   245                 250                 255
   Val Lys Glu Gly Met Ala Lys Asn Thr Tyr Gly Thr Gly Cys Phe Met
               260                 265                 270
   Leu Met Asn Thr Gly Glu Lys Ala Val Lys Ser Glu Asn Gly Leu Leu
           275                 280                 285
   Thr Thr Ile Ala Cys Gly Pro Thr Gly Glu Val Asn Tyr Ala Leu Glu
       290                 295                 300
   Gly Ala Val Phe Met Ala Gly Ala Ser Ile Gln Trp Leu Arg Asp Glu
   305                 310                 315                 320
   Met Lys Leu Ile Asn Asp Ala Tyr Asp Ser Glu Tyr Phe Ala Thr Lys
                   325                 330                 335
   Val Gln Asn Thr Asn Gly Val Tyr Val Val Pro Ala Phe Thr Gly Leu
               340                 345                 350
   Gly Ala Pro Tyr Trp Asp Pro Tyr Ala Arg Gly Ala Ile Phe Gly Leu
           355                 360                 365
   Thr Arg Gly Val Asn Ala Asn His Ile Ile Arg Ala Thr Leu Glu Ser
           370                 375                 380
   Ile Ala Tyr Gln Thr Arg Asp Val Leu Glu Ala Met Gln Ala Asp Ser
   385                 390                 395                 400
   Gly Ile Arg Leu His Ala Leu Arg Val Asp Gly Gly Ala Val Ala Asn
                   405                 410                 415
   Asn Phe Leu Met Gln Phe Gln Ser Asp Ile Leu Asp Thr Arg Val Glu
               420                 425                 430
   Arg Pro Glu Val Arg Glu Val Thr Ala Leu Gly Ala Ala Tyr Leu Ala
           435                 440                 445
   Gly Leu Ala Val Gly Phe Trp Gln Asn Leu Asp Glu Leu Gln Glu Lys
       450                 455                 460
   Ala Val Ile Glu Arg Glu Phe Arg Pro Gly Ile Glu Thr Thr Glu Arg
   465                 470                 475                 480
   Asn Tyr Arg Tyr Ala Gly Trp Lys Lys Ala Val Lys Arg Ala Met Ala
                   485                 490                 495
   Trp Glu Glu His Asp Glu
               500

## Beschreibung

Die Erfindung betrifft ein Verfahren zur fermentativen Herstellung von L-Aminosäuren, insbesondere L-Threonin, L-Tryptophan, L-Lysin, L-Valin und L-Homoserin, unter Verwendung von Glyzerin als Kohlenstoffquelle und mit Hilfe von Mikroorganismen der Familie Enterobacteriaceae oder der coryneformen Bakterien, die Allele des glpK-Gens besitzen, welche für Varianten der Glyzerinkinase kodieren.

### Stand der Technik

L-Aminosäuren finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, und von Stämmen coryneformer Bakterien, insbesondere Corynebacterium glutamicum (C. glutamicum) hergestellt werden. Wegen der großen Bedeutung wird ständig an der Verbesserung der Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch zum Beispiel Ionenaustauschchromatographie, oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie zum Beispiel das Threonin-Analogon α-Amino-β-Hydroxyvaleriansäure (AHV) oder auxotroph für regulatorisch bedeutsame Metabolite sind und die L-Aminosäuren wie zum Beispiel L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur Stammverbesserung L-Aminosäuren produzierender oder ausscheidender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden.

Zur Herstellung von L-Aminosäuren wird von der Fermentationsindustrie heute zumeist Glukose oder Saccharose als Kohlenstoffquelle eingesetzt. Es wird ständig nach neuen, preisgünstigen Rohstoffquellen gesucht.

Glyzerin (Propantriol) liegt in chemisch gebundener Form in Ölen und Fetten vor. Es verbindet als "Brücke" die Fettsäuremoleküle in den Triglyceriden. Bei der Herstellung von Treibstoff aus Rapsöl (Biodiesel, Rapsmethylester) entsteht als Kuppelprodukt (by-product) Glyzerin. Es steht somit der Fermentationsindustrie als Rohstoff in nennenswertem Umfang zur Verfügung.

Es ist bekannt, dass L-Threonin produzierende Stämme von Escherichia coli auf Glyzerin als alleiniger Kohlenstoffquelle wachsen (US-A-5,175,107). Zusammenfassende Darstellungen zum Glyzerinstoffwechsel (glycerol metabolism) von Enterobacteriaceae findet man bei Lin (In: Neidhardt (ed), Escherichia coli und Salmonella, American Society for Microbiology, Washington, D.C., USA: 307-342 (1996)).

Die Glyzerinkinase, die in ihrer enzymatisch aktiven Form mit dem GlpF-Fascilitator assoziiert vorliegt, phosphoryliert unter Verwendung von ATP cytoplasmatisches Glyzerin (Voegele et al., Journal of Bacteriology 175(4): 1087-94 (1993)) zu Glyzerin-3-Phosphat. Die Aktivität der Glyzerinkinase GlpK wird allosterisch durch Fruktose-1,6-Bisphosphat (FBP) und das Enzym IIA^{Glc}, dem Glukosespezifischen Phosphocarrier des Phosphoenolpyruvatabhängigen:Zucker-Phosphotransferase-Systems (PTS), gehemmt. Nach Holtman et al. (Journal of Bacteriology 183 : 3336-3344 (2001)) dominiert FBP die allosterische Kontrolle des Enzyms.

Die Nukleotidsequenz der Wildform des für die Glyzerinkinase GlpK von Escherichia coli kodierenden Gens wurde von Pettigrew et al. (Journal of Biological Chemistry 263 : 135-139 (1988)) bestimmt und ist in der Datenbank des National Center for Biotechnology Information (NCBI) der National Library of Medicin (Bethesda, MD, USA) unter der Zugangsnummer (accession number) U00096 (Region: 4113737-4115245) allgemein verfügbar.

Sie kann weiterhin der Patentanmeldung EP1715055 als Sequenz Nr. 15 entnommen werden.

In der EP1715055 ist eine Verbesserung der fermentativen Produktion von L-Aminosäuren durch rekombinante Mikroorganismen der Familie Enterobacteriaceae durch die Verstärkung des Gens glpK beschrieben.

Es sind glpK-Mutanten von E. coli bekannt, in denen die Inhibierung der Glyzerinkinase durch das Enzym IIA^{Glc} des PTS reduziert ist (Liu et al., Biochemistry 33 : 10120-10126 (1994); Pettigrew et al., Journal of Bacteriology 178 : 2846-2852 (1996); Pettigrew et al., Biochemistry 37 : 4875-4883 (1998)) bzw. die Mutation zu einer Aktivierung der Glyzerinkinase durch das Enzym IIA^{Glc} führt (Pawlyk und Pettigrew, Proceedings of the National Academy of Science USA 99(17):11115-11120 (2002)).

Darüberhinaus sind glpK-Mutanten von E. coli mit (1) erhöhter Glyzerinkinase-Aktivität bekannt, in denen (2) die Hemmung durch FBP reduziert ist (Herring et al., Nature Genetics 38(12): 1406-1412 (2006); Pettigrew et al., Journal of Bacteriology 178 : 2846-2852 (1996); Honisch et al., Genome Research 14 : 2495-2502 (2004)) und in denen (3) zusätzlich die Kontrolle der Glyzerin-Verwertung durch PTS-Zucker aufgehoben ist (Holtman et al., Journal of Bacteriology 183 : 3336-3344 (2001)).

Als PTS-Zucker bezeichnet man Zucker, welche durch das PTS-System aufgenommen werden. Diese hemmen die Aufnahme und die Verwertung gleichzeitig vorhandener nicht-PTS-Zucker bzw. nicht-PTS-Kohlenstoffquellen ("Katabolit-Repression"). Zu den PTS-Zuckern bei Enterobacteriacae gehören unter anderem Glukose, Saccharose, Fruktose, Trehalose, Maltose, Cellobiose, Mannose, Mannitol, N-Acetylglucosamin, β-Glukoside und Glucitol.

### Aufgabe der Erfindung

Die Erfinder haben sich die Aufgabe gestellt, neue Maßnahmen zur verbesserten fermentativen Herstellung von L-Aminosäuren, insbesondere L-Threonin, L-Tryptophan, L-Lysin, L-Valin und L-Homoserin, unter Verwendung von Glyzerin bereitzustellen.

### Beschreibung der Erfindung

Gegenstand der Erfindung ist ein Verfahren zur Herstellung von L-Aminosäuren, bevorzugt L-Threonin, L-Tryptophan, L-Lysin, L-Valin und L-Homoserin, dadurch gekennzeichnet, dass man folgende Schritte durchführt:
a) Fermentation von L-Aminosäure ausscheidenden rekombinanten Mikroorganismen der Familie Enterobacteriaceae oder der coryneformen Bakterien, welche ein glpK-Allel enthalten, das für ein Polypeptid mit Glyzerinkinase-Aktivität (ATP-abhängig) kodiert, dessen Aminosäuresequenz zu mindestens 95% identisch ist mit der von SEQ ID NO: 2, SEQ ID NO:4 oder SEQ ID NO:6, bevorzugt SEQ ID NO:2, wobei das Polypeptid eine Länge von 502 Aminosäuren besitzt und an Position 428 jede proteinogene Aminosäure, ausgenommen Glycin, bevorzugt L-Glutaminsäure, L-Asparaginsäure, L-Isoleucin, L-Histidin, L-Tryptophan und L-Phenylalanin, besonders bevorzugt L-Asparaginsäure, enthält,
   in einem Medium bei einer gewünschten Temperatur, wobei als Kohlenstoffquelle im Wesentlichen Glyzerin oder im Wesentlichen Glyzerin und einer oder mehrere der Zucker ausgewählt aus der Gruppe Glukose, Saccharose und Fruktose eingesetzt wird, und
b) Akkumulation der entstehenden L-Aminosäure in der Fermentationsbrühe.

Unter einer Glyzerinkinase versteht man ein Enzym welches die Phosphorylierung von Glyzerin zu Glyzerin-3-Phosphat katalysiert.

Unter proteinogenen Aminosäuren versteht man die Aminosäuren, die in natürlichen Proteinen, das heißt in Proteinen von Mikroorganismen, Pflanzen, Tieren und Menschen vorkommen. Hierzu gehören insbesondere L-Aminosäuren, ausgewählt aus der Gruppe L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin, L-Arginin und gegebenenfalls L-Selenocystein.

Die für die Maßnahmen der Erfindung eingesetzten Allele des glpK-Gens kodieren für Polypeptide mit der Aktivität einer Glyzerinkinase und mit einer Aminosäuresequenz, die zu mindestens 95 % oder mindestens 96 %, besonders bevorzugt zu mindestens 97 % oder mindestens 98 % und ganz besonders bevorzugt zu mindestens 99 % identisch sind mit SEQ ID NO:2, SEQ ID NO:4 oder SEQ ID NO:6, bevorzugt SEQ ID NO:2, und zusätzlich einen Aminosäureaustausch Position 428 enthalten. Die kodierten Polypeptide besitzen eine Länge entsprechend 502 Aminosäuren.

Für die Maßnahmen der Erfindung können auch Polynukleotide eingesetzt werden, die mit einem oder mehreren der Polynukleotide ausgewählt aus der Gruppe Polynukleotid komplementär zu SEQ ID NO:1, Polynukleotid komplementär zu SEQ ID NO:3 und Polynnukleotid komplementär zu SEQ ID NO:5, bevorzugt Polynukleotid komplementär zu SEQ ID NO:1, hybridisieren und für Polypeptide mit Glyzerinkinase Aktivität kodieren, welche an Position 428 jede proteinogene Aminosäure, ausgenommen Glycin, bevorzugt L-Glutaminsäure, L-Asparaginsäure, L-Isoleucin, L-Histidin, L-Tryptophan und L-Phenylalanin, besonders bevorzugt L- Asparaginsäure, besitzen. Die kodierten Polypeptide besitzen eine Länge entsprechend 502 Aminosäuren.

Anleitungen zur Hybridisierung von DNA-Sequenzen findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% oder mindestens 99,9% Identität zur Sequenz der eingesetzten Sonde bzw. zu den in SEQ ID NO: 1, SEQ ID NO: 3 oder SEQ ID NO: 5 dargestellten Nukleotidsequenzen besitzen. Weitere Anleitungen zur Hybridisierung sind in Form sogenannter Kits am Markt erhältlich (zum Beispiel DIG Easy Hyb von der Firma Roche Diagnostics GmbH, Mannheim, Deutschland, Catalog No. 1603558).

Für die Maßnahmen der Erfindung werden weiterhin Allele des glpK-Gens eingesetzt, die für Polypeptide mit Glyzerinkinase Aktivität kodieren, welche die Aminosäuresequenz von SEQ ID NO:2, SEQ ID NO:4 oder SEQ ID NO:6, bevorzugt SEQ ID NO:2, umfassen oder besitzen mit dem angegebenen Aminosäureaustausch an Position 428 und mit einem oder mehreren der Merkmale ausgewählt aus der Gruppe
a) ein bis zehn konservative Aminosäureaustausche,
b) eine Verlängerung am C-Terminus um eine (1) bis zwanzig Aminosäure(n).

Es ist selbsterklärend, dass die unter a) erwähnten konservativen Aminosäureaustausche nicht den beschriebenen Aminosäureaustausch an Position 428 betreffen.

Die Anzahl der konservativen Aminosäureaustausche beträgt bevorzugt höchstens fünf (5), besonders bevorzugt höchstens vier (4) und ganz besonders bevorzugt höchstens drei (3), oder höchstens zwei (2).

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Bevorzugt beträgt die Verlängerung am C-Terminus des Polypeptids nicht mehr als 10, 5, 3 oder 2 Aminosäuren.

An Position 428 wird der Austausch des Glycin gegen L-Glutaminsäure (G428E), L-Asparaginsäure (G428D), L-Isoleucin (G428I), L-Histidin (G428H), L-Tryptophan (G428W) und L-Phenylalanin (G428F) bevorzugt. Besonders bevorzugt wird der Austausch gegen L- Asparaginsäure (G428D).

Bei den für die Massnahmen der Erfindung eingesetzten Mikroorganismen handelt es sich um, insbesonders rekombinante, L-Aminosäure ausscheidende Vertreter der Familie Enterobacteriaceae oder der coryneformen Bakterien. Die Vertreter der Enterobacteriaceae werden bevorzugt ausgewählt aus den Gattungen Escherichia, Erwinia, Providencia und Serratia. Die Gattungen Escherichia und Serratia werden besonders bevorzugt. Bei der Gattung Escherichia ist insbesondere die Art Escherichia coli und bei der Gattung Serratia insbesondere die Art Serratia marcescens zu nennen.

Unter den coryneformen Bakterien wird die Gattung Corynebacterium bevorzugt. Besonders bevorzugt sind Stämme, die auf folgenden Arten beruhen: Corynebacterium efficiens, Corynebacterium glutamicum und Corynebacterium ammoniagenes, wobei die Art Corynbacterium glutamicum ganz besonders bevorzugt wird.

Einige Vertreter der Art Corynebacterium glutamicum sind im Stand der Technik auch unter anderen Artbezeichnungen wie zum Beispiel Corynebacterium acetoacidophilum, Corynebacterium lilium, Corynebacterium melassecola, Brevibacterium flavum, Brevibacterium lactofermentum, und Brevibacterium divaricatum ATCC14020. Der Begriff "Micrococcus glutamicus" für Corynebacterium glutamicum war ebenfalls gebräuchlich.

Zur Herstellung der L-Aminosäure-ausscheidenden Stämme der Familie Enterobacteriaceae oder der coryneformen Bakterien, welche glpK-Allele enthalten, die für die angegebenen Varianten der Glyzerinkinase kodieren, werden bevorzugt Stämme verwendet (Ausgangsstämme bzw. Elternstämme), die bereits die Fähigkeit besitzen, die gewünschte L-Aminosäure in der Zelle anzureichern und/oder in das sie umgebende Nährmedium auszuscheiden bzw. in der Fermentationsbrühe zu akkumulieren. Hierfür kann auch der Ausdruck "produzieren" verwendet werden. Insbesondere besitzen die für die Maßnahmen der Erfindung eingesetzten Stämme die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l an L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten L-Aminosäuren-ausscheidenden Stämme produzieren eine oder mehrere, bevorzugt eine oder im Wesentlichen eine Aminosäure ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin, bevorzugt ausgewählt aus der Gruppe L-Threonin, L-Tryptophan, L-Lysin, L-Valin und L-Homoserin. Der Begriff L-Aminosäure oder Aminosäure umfasst auch deren Salze.

Der Begriff" eine oder im Wesentlichen eine Aminosäure" berücksichtigt, dass zusätzlich zu der gewünschten L-Aminosäure eine oder mehrere weitere der genannten L-Aminosäuren (Nebenaminosäuren) produziert werden können. Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als Elternstamm geeignete, L-Threonin produzierende bzw. ausscheidende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4,278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660)

Als Elternstamm geeignete, L-Threonin produzierende bzw. ausscheidende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992)).

L-Threonin produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164 (pGH5) (WO94/08031)
- E. coli AGX17 (pGX44) (NRRL B-12263) (US4,371,614)
- E. coli AGX6 (pGX50) aroP (NRRL B-12264) (US4,371,614)
- Escherichia coli AGX17/pGX50, pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5,939,295).

L-Tryptophan produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phophoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Als Elternstamm geeigneter, L-Homoserin ausscheidender bzw. produzierender Stamm der Gattung Escherichia, insbesondere der Art Escherichia coli ist beispielsweise zu nennen:
Escherichia coli NZ10rhtΔ23/pAL4 (US 6,960,455).

L-Homoserin produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe: Bedürftigkeit für L-Threonin, Bedürftigkeit für L-Methionin, Bedürftigkeit für L-Isoleucin, eine defekte Homoserin-Kinase, gegebenenfalls Fähigkeit zur Saccharose-Verwertung, Verstärkung der Homoserin-Dehydrogenase I/Aspartatkinase I, bevorzugt der feed back resistenten Form, Verstärkung des RhtA-Genproduktes.

Als Elternstamm geeignete, L-Lysin ausscheidende bzw. produzierende Stämme der Gattung Escherichia, insbesondere der Art Escherichia coli sind beispielsweise zu nennen:
- Escherichia coli pDA1/TOC21R (= CNCM I-167) (FR-A-2511032),
- Escherichia coli NRRL B-12199 (US4,346,170)
- Escherichia coli NRRL B-12185 (US4,346,170).

Als Elternstamm geeigneter, L-Valin ausscheidender bzw. produzierender Stamm der Gattung Escherichia, insbesondere der Art Escherichia coli ist beispielsweise zu nennen:
- Escherichia coli AJ11502 (NRRL B-12288) (US-A-4391907).

Als Elternstamm geeignete, L-Lysin ausscheidende bzw. produzierende Stämme der Gattung Corynebacterium, insbesondere der Art Corynebacterium glutamicum sind beispielsweise zu nennen:
- Corynebacterium glutamicum DM58-1/pDM6 (= DSM4697) beschrieben in EP 0 358 940,
- Corynebacterium glutamicum MH20-22B (= DSM16835) beschrieben in Menkel et al. (Applied and Environmental Microbiology 55(3), 684-688 (1989)),
- Corynebacterium glutamicum AHP-3 (= Ferm BP-7382) beschrieben in EP 1 108 790, und
- Corynebacterium glutamicum NRRL B-11474 beschrieben in US 4,275,157.

Als Elternstamm geeignete, L-Valin ausscheidende bzw. produzierende Stämme der Gattung Corynebacterium, insbesondere der Art Corynebacterium glutamicum sind beispielsweise zu nennen:
- Corynebacterium glutamicum FERM BP-3006 beschrieben in US 5,521,074, und
- Corynebacterium glutamicum FERM BP-1764 beschrieben in US 5,188,948.

Bei den der Erfindung zugrunde liegenden Arbeiten wurde gefunden, dass L-Aminosäure produzierende Mikroorganismen der Familie Enterobacteriaceae nach Austausch des WildtypglpK-Gens gegen ein Allel des glpK-Gens, welche für eine Variante der ATP-abhängigen Glyzerinkinase kodiert, den Aminosäureaustausch G428D enthält, in erhöhtem Umfang L-Aminosäuren, insbesondere L-Threonin, L-Tryptophan, L-Lysin, L-Valin und L-Homoserin, produzieren.

Bei den der Erfindung zugrunde liegenden Arbeiten wurde weiterhin gefunden, dass L-Aminosäure produzierende Mikroorganismen coryneformer Bakterien nach Expression eines Allel des glpK-Gens, welches für eine Variante der ATP-abhängigen Glyzerinkinase kodiert, die den Aminosäureaustausch G428D enthält, in erhöhtem Umfang L-Aminosäuren produzieren.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden.

Das glpK-Gen von Escherichia coli K12 kodiert für eine ATPabhängige Glyzerinkinase (GlpK, EC-Nr.: 2.7.1.30). Die Nukleotidsequenz des Wildtypgens ist in den öffentlichen Datenbanken unter der Zugangsnummer (Accession No.) U00096 (Region: 4113737-4115245) verfügbar. Es ist im Stand der Technik auch unter dem alternativer Gennamen b3926 bekannt. Das kodierte Polypeptid besitzt eine Länge von 502 Aminosäuren. Nach erfolgter Translation wird das N-terminale Methionin abgespalten.

Von den ebenfalls zur Familie Enterobacteriaceae gehörenden Arten Salmonella typhimurium (Accession No.: NC 003197 (Sequenz des gesamten Genoms)) und Shigella flexneri (Accession No.: NC 004337 (Sequenz des gesamten Genoms)) ist die Nukleotidsequenz für das Wildtyp glpK-Gen ebenso bekannt.

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber ist die bekannte Sequenz zum glpK-Gen des Wildtyps von Escherichia coli unter der SEQ ID NO: 1 und die ebenfalls bekannten Sequenzen zum glpK-Gen des Wildtyps von Salmonella typhimurium bzw. Shigella flexneri unter der SEQ ID NO: 3 bzw. SEQ ID NO: 5 dargestellt. Die von diesen Leserahmen kodierten Proteine sind als SEQ ID NO: 2, SEQ ID NO: 4 und SEQ ID NO: 6 dargestellt.

Es ist bekannt, dass durch wirtseigene Enzyme (Methionin-Aminipeptidase) die N-terminale Aminosäure Methionin abgespalten werden kann. Die Positionsangaben für die jeweiligen Aminosäurereste verschieben sich dementsprechend. Beispielsweise rückt die L-Asparaginsäure von Position 11 des kodierten Polypeptids (Siehe SEQ ID NO:2) nach Entfernung des N-terminalen Methionins an Position 10.

Als offener Leserahmen (ORF, open reading frame) wird ein Abschnitt einer Nukleotidsequenz bezeichnet, der für ein Protein beziehungsweise ein Polypeptid oder eine Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Ein Gen oder Allel ist chemisch ein Polynukleotid. Ein anderer Begriff hierfür ist Nukleinsäure, insbesondere Desoxyribonukleinsäure.

Die Begriff Polypeptid und Protein sind gegenseitig austauschbar.

Als Genprodukt bezeichnet man im Allgemeinen das von einer Nukleotidsequenz, d.h. einem ORF, einem Gen oder einem Allel kodierte Protein oder die kodierte Ribonukleinsäure.

Zur Erzeugung der glpK-Allele, die in den für das erfindungsgemäße Verfahren eingesetzten Mikroorganismen zum Einsatz kommen, werden unter anderem im Stand der Technik beschriebene Methoden zur gerichteten Mutagenese verwendet.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Zur Konstruktion von Punktmutationen im glpK-Gen wird zum Beispiel das Quick Change Site-Directed Mutagenesis Kit der Firma Stratagene (Amsterdam, Niederlande) verwendet. Bei Verwendung dieser Methoden wird das im Stand der Technik beschriebene glpK-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden.

Die erzeugten glpK-Allele können beispielsweise durch Transformation und das Verfahren des Gen- bzw. Allelaustausches in geeignete Stämme eingebaut werden.

Eine bei Enterobacteriacae gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pKO3 (Link et al., Journal of Bacteriology 179 : 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten glpK-Allele durch Konjugation oder Transduktion in verschiedene Stämme zu überführen.

Die Mutationen von glpK können ebenfalls in Expressionsplasmiden durchgeführt werden, so dass die resultierenden Proteine überproduziert werden können.

In einem erfindungsgemäßen Verfahren können die glpK-Allele, die für die aufgeführten Glyzerinkinasevarianten kodieren auch überexprimiert werden.

Methoden zur Überführung und Expression von Polynukleotiden in coryneforme Bakterien sind im Stand der Technik hinlänglich bekannt.

So sind im Stand der Technik zahlreiche Promotoren beschrieben, die es ermöglichen eine gewünschte Konzentration bzw. Aktivität der jeweiligen Glyzerinkinasevariante einzustellen. Beispielsweise kann der lysC-Promotor der Mutante DM58-1, der bei Kalinowski et al. (Molecular Microbiology 5(5), 1197-1204 (1991) beschrieben ist, oder der gap-Promotor, der in der Patentanmeldung mit der Anmeldenummer EP 06007373.1 beschrieben ist, eingesetzt werden. Weiterhin können die in der Patentanmeldung mit der Anmeldenummer EP 06117294.6 beschrieben Promotoren, die in der Zeitschrift Research Disclosure (Beitrag 512057 in der Ausgabe vom Dezember 2006, Seite 1616 bis 1618) beschriebenen Promotoren, die von Patek et al. (Journal of Biotechnology 104(1-3), 311-323 (2003) beschriebenen Promotoren oder die von Vasicova et al. (Journal of Bacteriology 181, 6188-6191 (1999)) beschriebenen Varianten des dapA-Promotors, beispielsweise der Promotor A25 eingesetzt werden.

In gleicher Weise können auch die aus der Escherichia coli Genetik bekannten Promotoren wie beispielsweise tac-Promotor, trp-Promotor, trc-Promotor und lpp-Promotor oder der P_{L}- und P_{R}-Promotor des Phagen λ eingesetzt werden.

Das dergestalt mit einem Promotor versehene Polynukleotid kann in Form einer (1) oder mehrerer Kopien in das gewünschte coryneforme Bakterium eingebaut werden.

Hierzu können beispielsweise Plasmide verwendet werden die von coryneformen Bakterium repliziert werden. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al. (Journal of Biotechnology 104, 27-40 (2003)).

Weiterhin kann eine Kopie des Polynukleotids in das Chromosom eines coryneformen Bakteriums eingefügt werden.

In einer derartigen Ausführungsform wird ein in coryneformen Bakterien nicht replikatives Plasmid verwendet. Hierbei wird am 5'-Terminus und am 3'-Terminus des gewünschten Gens, kodierend für ein Polypeptid mit Glyzerinkinase Aktivität, ein DNA-Fragment angefügt, welches den Zielort innerhalb des Chromosoms des coryneformen Bakteriums darstellt, in den das entsprechende Gen durch homologe Rekombination integriert wird. Geeignete Zielorte für Corynebacterium glutamicum sind unter anderem in der Tabelle 3 der WO 03/040373, in den Tabellen 12 und 13 der WO 04/069996, bei Aham et al. (Applied and Environmental Microbiology 67(12), 5425-5430 (2001), oder Correia et al. (FEMS Microbiology Letters 142, 259-264 (1996)) beschrieben. Nach Konjugation oder Transformation und homologer Rekombination mittels mindestens zweier Rekombinationsererignisse enthält der resultierende Stamm mindestens zwei Kopien des betreffenden Gens.

Weiterhin kann es für die Produktion von L-Aminosäuren vorteilhaft sein, zusätzlich zum Einsatz eines der angegebenen glpK-Allele, ein oder mehrere Enzyme des jeweiligen spezifischen Biosyntheseweges zu verstärken.

So kann es für die Produktion von L-Threonin mit Stämmen der Familie Enterobacteriaceae vorteilhaft sein, zusätzlich zum Einsatz eines der angegebenen glpK-Allele, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

So kann es für die Produktion von L-Trytophan mit Stämmen der Familie Enterobacteriaceae vorteilhaft sein, zusätzlich zum Einsatz eines der angegebenen glpK-Allele, ein oder mehrere Enzyme des bekannten Tryptophan-Biosyntheseweges oder Enzyme der Serin-Biosynthese oder Enzyme für die Produktion von 3-deoxy-D-arabino-heptulosonat-7-Phosphat (DHAP) und Chorismat zu verstärken. Die Verwendung endogener Gene wird im Allgemeinen bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Rekombinante Mikroorganismen mit einer solchen Verstärkung werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch eine verstärkende Mutation aus der eigenen, upstream des Gens oder ORFs befindlichen regulatorischen Sequenz hervorgegangenen Promotor handeln, oder es wirde ein geeigneter bzw. effizienter Promotor mit dem Gen oder ORF fusioniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die Maßnahmen der Verstärkung durchgeführt werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin- und L-Tryptophan-Produktion zu steigern, des weiteren kann auch die Verwendung von Promotoren zur Genexpression, die eine andere zeitliche Genexpression ermöglicht, vorteilhaft sein. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die ORFs, Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren oder der nar-Promotor verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Eine andere zeitliche Genexpression kann beispielsweise wie bei Walker et al. (Journal of Bacteriology 181: 1269-80 (1999)) beschrieben durch die Verwendung des Wachstumsphasen-abhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden.

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie zum Beispiel von pACYC184 abgeleitete Kloniervektoren (Bartolome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Vektor, bevorzugt Plasmidvektor, transformierten Stamm einsetzen, wobei der Vektor mindestens eines oder mehrere der im folgenden genannten Gene oder für deren Genprodukte kodierende Nukleotidsequenzen oder Allele trägt.

So können beispielsweise für die Produktion von L-Threonin, gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992)),
- das für die Phosphoenolpyruvat-Carboxylase kodierende ppc-Gen (WO 02/064808),
- die für die Untereinheiten der Transhydrogenase kodierenden Gene pntA und pntB (European Journal of Biochemistry 158: 647-653 (1986)),
- das für das Threoninresistenz vermittelnde Protein kodierende Gen rhtC
   (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983)),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glukose-spezifische IIA Komponente des Phosphotransferase-Systems PTS kodierende crr-Gen des ptsHIcrr-Operons (WO 03/004674),
- das für die Glukose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yibD von Escherichia coli (Accession Number AE000439 des National Center for Biotechnology Information (NCBI, Bethesda, MD, USA), WO 05/075627),
verstärkt, insbesondere überexprimiert werden.

Desweiteren können beispielsweise für die Produktion von L-Tryptophan, gleichzeitig eines oder mehrere der Gene, ausgewählt aus der Gruppe
- mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
- das für die 3-Phosphoglycerat-Dehydrogenase kodierende serA-Gen (WO87/01130)
- das für die Phosphoserinphosphatase kodierende serB-Gen (WO87/01130)
- das für die Phosphoserinaminotransferase kodierende serC-Gen (WO87/01130)
- das für die L-Tyrosin sensitive DHAP-Synthase kodierende aroF-Gen (WO87/01130; EP1270721)
- das für die L-Phenylalanin sensitive DHAP-Synthase kodierende aroG-Gen (WO87/01130; EP1270721)
- das für die L-Tryptophan sensitive DHAP-Synthase kodierende aroH-Gen (WO87/01130; EP1270721)
- das für die Phosphoenolpyruvat-Synthase kodierende pps-Gen (WO96/08567)
- das für die Phosphoenolpyruvat-Carboxykinase kodierende pckA-Gen (WO2004/090125),
- das für die Transketolase A kodierende tktA-Gen (US5,168,056),
- das für die Transketolase B kodierende tktB-Gen (US5,168,056),
- das Genprodukt des offenen Leserahmens (ORF) yddG von Escherichia coli (Accession Number NC000913 (Region 1544312-1545193) des National Center for Biotechnology Information (NCBI, Bethesda, MD, USA), EP1449918),
verstärkt, insbesondere überexprimiert werden.

Desweiteren können zur Herstellung der L-Aminosäuren Mikroorganismen der Familie Enterobacteriaceae fermentiert werden, in denen man zur Optimierung des Glyzerin-Stoffwechsels zusätzlich gleichzeitig eines oder mehrere der Gene, ausgewählt aus der Gruppe (EP1715055):
- das für die große Untereinheit der sn-Glyzerin-3-Phosphat-Dehydrogenase (anaerob) kodierende glpA-Gen,
- das für die Untereinheit zur Membranverankerung der sn-Glyzerin-3-Phosphat-Dehydrogenase (anaerob) kodierende glpB-Gen,
- das für die kleine Untereinheit der sn-Glyzerin-3-Phosphat-Dehydrogenase (anaerob) kodierende glpC-Gen,
- das für die Glyzerin-3-Phosphat-Dehydrogenase (aerob) kodierende glpD-Gen,
- das für die Schwefeltransferase kodierende glpE-Gen,
- das für den Glyzerinfascilitator GlpF kodierende glpF-Gen,
- das glpG-Gen
- das für die periplasmatische Glyzerinphosphodiesterase kodierende glpQ-Gen,
- das für die Glyzerin-3-Phosphat-Permease kodierende glpT-Gen,
- das für die Fruktose-1,6-Bisphosphatase II kodierende glpX-Gen,
- das für die Triosephosphat-Isomerase kodierende tpiA-Gen,
- das für die Glyzerin-Dehydrogenase (NAD-abhängig) kodierende gldA-Gen,
- das für die N-terminale Domäne der Dihydroxyaceton-Kinase kodierende dhaK-Gen,
- das für die C-terminale Domäne der Dihydroxyaceton-Kinase kodierende dhaL-Gen,
- das für die PTS-Protein-Untereinheit der Dihydroxyaceton-Kinase kodierende dhaM-Gen,
- das für den Aktivator des dha-Operons kodierende dhaR-Gen,
- das für die Fruktose-6-Phosphat-Aldolase I kodierende fsa-Gen, und
- das für die Fruktose-6-Phosphat-Aldolase II kodierende talC-Gen
verstärkt, insbesondere überexprimiert.

Desweiteren können zur Herstellung der L-Aminosäuren Mikroorganismen der Familie Enterobacteriaceae fermentiert werden, in denen man zur Optimierung des Glyzerin-Stoffwechsels zusätzlich gleichzeitig das für den Repressor des glp-Regulons kodierende glpR-Gen (EP1715056) abschwächt, insbesondere ausschaltet oder die Expression verringert.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die Maßnahmen der Abschwächung durchgeführt werden.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Eine Verringerung der Genexpression kann auch durch den Einbau einer Stop-Kodon-Mutation in den Kodierbereich eines gewünschten Gens bei gleichzeitiger Suppression der Stop-Kodon-Mutation durch einen geeigneten t-RNA-Suppressor erzielt werden. Diese Vorgehensweise ist in der WO 03/074719 beschrieben.

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie zum Beispiel dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die genannten Maßnahmen werden vorzugsweise im 5'-terminalen Teil der Kodierregion durchgeführt, welcher für den N-Terminus des Polypeptids kodiert. Bezeichnet man die Gesamtlänge eines Polypeptids (gemessen als Anzahl der chemisch verbundenen L-Aminosäuren) mit 100%, so gehören - im Rahmen der Abschwächungsmaßnahmen der vorliegenden Erfindung - zum N-Terminus des Polypeptids der Teil der Aminosäuresequenz, welcher, gerechnet ab der Start-Aminosäure L-Formyl-Methionin 80% der nachfolgenden L-Aminosäuren enthält.

Anleitungen zur Erzeugung derartiger Mutationen gehören zum Stand der Technik und können bekannten Lehrbüchern der Genetik und Molekularbiologie wie zum Beispiel dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Threonin vorteilhaft sein, zusätzlich zum Einsatz eines der angegebenen glpK-Allele, eines oder mehrere der Gene ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das Genprodukt des offenen Leserahmens (ORF) yjfA von Escherichia coli (Accession Number AAC77180 des National Center for Biotechnology Information (NCBI, Bethesda, MD, USA), WO 02/29080),
- das Genprodukt des offenen Leserahmens (ORF) ytfP von Escherichia coli (Accession Number AAC77179 des National Center for Biotechnology Information (NCBI, Bethesda, MD, USA), WO 02/29080),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fruktose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist, und
- das für die Aspartat Ammonium-Lyase kodierende aspA-Gen (WO 03/008603)

abzuschwächen, insbesondere auszuschalten oder die Expression zu verringern. Diese Massnahmen werden gegebenenfalls zusätzlich zu bzw. in geeigneter Kombination mit den angegebenen Massnahmen zur Verstärkung der Threonin-Produktion und den Massnahmen zur Optimierung des Glyzerin-Stoffwechsels durchgeführt.

Weiterhin kann es für die Produktion von L-Tryptophan vorteilhaft sein, zusätzlich zum Einsatz eines der angegebenen glpK-Allele, eines oder mehrere der Gene ausgewählt aus der Gruppe
- das für die Tryptophanase kodierende tnaA-Gen (US4,371,614),
- das für den Repressor des trp-Operons kodierende trpR-Gen (US4,371,614)
- das für die Chorismat-Mutase T und Prephenat-Dehydrogenase kodierende tyrA-Gen (US4,371,614),
- das für die Chorismat-Mutase P und Prephenat-Dehydrogenase kodierende pheA-Gen (US4,371,614),
- das für das Tryptophan spezifische Transportprotein kodierende mtr-Gen (US5,756,345),
- das für die Tryptophan-Permease kodierende tnaB-Gen (US5,756,345),
- das für den Transporter für aromatische Aminosäuren kodierende aroP-Gen (US5,756,345),
- das für die L-Serin Deaminase kodierende sdaA-Gen (EP0149539),
- das für die Glukose-6-Phosphat-Isomerase kodierende pgi-Gen (WO87/01130),
- das für die Tyrosin-Aminotransferase kodierende tyrB-Gen (WO87/01130)

abzuschwächen, insbesondere auszuschalten oder die Expression zu verringern. Diese Massnahmen werden gegebenenfalls zusätzlich zu bzw. in geeigneter Kombination mit den angegebenen Massnahmen zur Verstärkung der Tryptophan-Produktion und den Massnahmen zur Optimierung des Glyzerin-Stoffwechsels durchgeführt.

Weiterhin kann es für die Produktion von L-Aminosäuren, vorteilhaft sein, zusätzlich zum Einsatz eines der angegebenen glpK-Allele, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982) .

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch-Verfahren (Satzkultivierung), im fed batch-Verfahren (Zulaufverfahren), im repeated fed batch-Verfahren (repetitives Zulaufverfahren) oder in einem kontinuierlichen Verfahren kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium bzw. Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Das Kulturmedium muss weiterhin Salze von Metallen enthalten, wie zum Beispiel Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden.

Als Kohlenstoffquelle werden im Rahmen dieser Erfindung Verbindungen verstanden, die lediglich Kohlenstoff, Sauerstoff und Wasserstoff im Molekül enthalten und von den Mikrorganismen zur Bildung ihrer Biomasse (Zellmasse) und zur Produktion der L-Aminosäuren genutzt werden. In einem erfindungsgemäßen Verfahren besteht die Kohlenstoffquelle in einem Aspekt im Wesentlichen aus Glyzerin und in einem weiteren Aspekt im Wesentlichen aus Glyzerin und einem oder mehreren der Zucker ausgewählt aus der Gruppe Glukose, Saccharose, Fruktose, Trehalose, Maltose, Cellobiose, Mannose, Mannitol, N-Acetylglucosamin, β-Glucoside und Glucitol, bevorzugt im Wesentlichen aus einem oder mehreren der Zucker ausgewählt aus der Gruppe Glukose, Saccharose und Fruktose.

Der Begriff "im Wesentlichen" berücksichtigt in diesem Zusammenhang zum einen die Tatsache, dass in industriellen Fermentationsprozessen zur Herstellung von L-Aminosäuren nicht nur chemisch reine Einsatzstoffstoffe sondern auch ggf. verunreinigte Einsatzstoffstoffe von technischer oder geringerer Qualität verwendet werden. Weiterhin berücksichtigt der Begriff die Zusammensetzung typischer komplexer, mikrobiologischer Medienbestandteile, die chemische Veränderung der Einsatzstoffe infolge der Sterilisation durch Hitze, und schließlich die sich durch die Beimpfung eines Nährmediums mittels einer Vorkultur (eines Inokulums) ergebende Übertragung von Medienbestandteilen oder Bestandteilen der Fermentationsbrühe.

In einem erfindungsgemäßen Verfahren kann nicht nur chemisch reines Glyzerin sondern auch Glyzerin von technischer Qualität (Rohglyzerin) verwendet werden. Glyzerin von technischer Qualität enthält geringe Konzentrationen von Verbindungen, die im Herstellprozess angefallen sind und nicht vollständig abgetrennt wurden. Hierzu gehören unter anderem Wasser, verschiedene Salze und organische Verbindungen.

Rohglyzerin, das aus Öl vom Raps gewonnen wird, enthält beispielsweise als Verunreinigungen Verbindungen wie Linolsäure, Linolensäure, Ölsäure und deren Methylester oder auch Glyzerinmonoessigsäure, Mono-, Di- und Triglyceride, die gegebenenfalls als Kohlenstoffquelle verwendet werden können. Der Gehalt an derartigen als Kohlenstoffquelle dienenden Verbindungen beträgt im Allgemeinen ≤ (höchstens) 7,5 Gew.-%, bevorzugte ≤ 5 Gew.-%, besonders bevorzugt ≤ 2,5 Gew.-% (bezogen auf das wasserfreie Rohglyzerin). Das für die Massnahmen der Erfindung eingesetzte Glyzerin hat einen Gehalt von ≥ (mindestens) 90 Gew.-%, bevorzugt ≥ 92,5 Gew.-% und besonders bevorzugt ≥ 95 Gew.-% (bezogen auf den wasserfreien Einsatzstoff).

Eine wichtige Quelle von Glukose sind Hydrolysate von Stärke. Typischerweise Begleitsubstanzen sind dementsprechend beispielsweise Maltose und/oder Isomaltose in Konzentrationen von ungefähr 0,1 bis 2 Gew.-%. Die für die Massnahmen der Erfindung verwendete Glukose hat einen Gehalt von ≥ (mindestens) 90 Gew.-%, bevorzugt ≥ 95 Gew.-% (bezogen auf die Trockensubstanz).

Es ist bekannt, dass in Lösungen von Saccharose die Saccharose bei erhöhter Temperatur, wie sie beispielsweise bei der Hitzesterilisation gegeben ist, zu einem geringen Anteil zu Glukose und Fruktose invertiert. In der für die Massnahmen der Erfindung eingesetzten Saccharose bzw. Saccharoselösung beträgt der Anteil an Glukose und Fruktose ≤ (maximal) 10 Gew.-%, bevorzugt ≤ 5 Gew.-% (bezogen auf die Trockensubstanz).

Die bei der Herstellung von Saccharose aus Zuckerrüben anfallende Melasse (Rübenmelasse) enthält ein Zuckergemisch, das im Wesentlichen aus Saccharose besteht.

In der Fachwelt sind beispielsweise unter der Bezeichnung "Isosirup", "high Fruktose corn sirup" oder "fruktosehaltiger Glukosesirup" konzentrierte Lösungen enthaltend Glukose und Fruktose bekannt. Diese werden durch Hydrolyse von Stärke mit anschließender Behandlung der Glukose mit Isomerase hergestellt. Sie enthalten ein Zuckergemisch, das im Allgemeinen ungefähr 50 - 55 Gew.-% Glukose und 40 - 45 Gew.-% Fruktose enthält (bezogen auf die Trockensubstanz). Aufgrund des Herstellungsprozesses enthalten derartige Sirupe unter anderem im Allgemeinen Maltose in einer Konzentration von maximal ungefähr 6 Gew.-% (bezogen auf die Trockensubstanz).

Konzentrierte Lösungen, die im Wesentlichen ein Zuckergemisch enthalten, das aus equimolaren Anteilen von Glukose und Fruktose besteht, sind ebenfalls in der Fachwelt bekannt. Diese werden beispielsweise durch Behandlung von Saccharose mit Invertase hergestellt und enthalten dementsprechend geringe Anteile von Saccharose (≤ 5 Gew.-% bezogen auf die Trockensubstanz).

In der Fachwelt sind weiterhin konzentrierte Lösungen enthaltend ein Zuckergemisch bestehend aus Saccharose, Glukose und Fruktose bekannt. Diese werden durch partielle Invertierung von Saccharose hergestellt. Ein bekanntes Konzentrat besteht aus 38,5 Gew.-% Saccharose, 38,5 Gew.-% Invertzucker (d. h. Glukose und Fruktose im Verhältnis 1 : 1) und 23 Gew.-% Wasser.

Die bei der Herstellung von Saccharose aus Zuckerrohr anfallende Melasse (Zuckerrohrmelasse) enthält ein Zuckergemisch, das im Wesentlichen aus Saccharose, Glukose und Fruktose besteht.

Der Begriff "im Wesentlichen" berücksichtigt weiterhin die Tatsache, dass in Fermentationsmedien eingesetzte komplexe Medienbestandteile wie beispielsweise Hefeextrakt oder Maisquellwasser in variierenden Anteilen Verbindungen enthalten, die als Kohlenstoffquelle dienen können. So enthält Hefeextrakt unter anderem Trehalose und/oder Glukose. Maisquellwasser (corn steep liquor) enthält ungefähr 10 bis 20 Gew.-% (bezogen auf die Trockensubstanz) Milchsäure. Derartige komplexe Medienbestandteile werden bei Bedarf im Allgemeinen in einer Konzentration von 0,5 bis 20 g/l und auch in höheren Konzentrationen im Fermentationsmedium eingesetzt.

Informationen zur Herstellung von Glukose, Saccharose und Fruktose und zur Zusammensetzung komplexer Medienbestandteile findet man unter anderem in den. Lehrbüchern von Bartens (Zuckertechnologie, Rüben und Rohrzuckerherstellung, Verlag Dr. Albert Bartens KG, Berlin (Deutschland)), McGinnis (Beet-Sugar Technology, third edition, Studio of Printcraft, Fort Collins, Colorado, US (1982)), Drews (Mikrobiologisches Praktikum, 3. Auflage, Springer Verlag, Berlin (Deutschland), 1976), Rehm (Industrielle Mikrobiologie, 2. Auflage, Springer Verlag, Berlin (Deutschland), 1980) und Crueger und Crueger (Lehrbuch der Angewandten Mikrobiologie, Akademische Verlagsgesellschaft, Wiesbaden (Deutschland), 1982)

Der Begriff "im Wesentlichen" berücksichtigt schließlich auch die Tatsache, dass durch Beimpfung eines Nährmediums unter Verwendung einer sogenannten Vorkultur (Inokulum) nicht verbrauchte oder während der Vorkultur gebildete Kohlenstoffquellen in die für die Herstellung der jeweiligen L-Aminosäure verwendeten Kultur übertragen werden. Der Anteil der Vorkultur in der durch die Inokulierung entstehenden Kultur beträgt im Allgemeinen 2 bis 20 % ggf. auch maximal 50 %.

In einem erfindungsgemäßen Verfahren besteht das eingesetzte Glyzerin oder Rohglyzerin im Allgemeinen zu ≥ (mindestens) 90 Gew.-%, bevorzugt zu mindestens 92,5 Gew.-% und besonders bevorzugt zur ≥ 95 Gew.-% aus Glyzerin (bezogen auf den wasserfreien Einsatzstoff). Glyzerin mit einer Reinheit von ≥ 97 Gen.-%, ≥ 98 Gew.-% oder ≥ 99 Gew.-% kann ebenfalls eingesetzt werden.

In einem erfindungsgemäßen Verfahren besteht der bevorzugt eingesetzte Zucker, bzw. bestehen die bevorzugt eingesetzten Zuckermischungen im Allgemeinen zu ≥ (mindestens) 90 Gew.-%, gegebenenfalls bevorzugt ≥ 95 Gew.-%, aus einem oder mehreren der angegebenen Zucker ausgewählt aus der Gruppe Glukose, Saccharose und Fruktose (bezogen auf die Trockensubstanz).

Werden in einem erfindungsgemäßen Verfahren Mischungen aus Glyzerin und Glukose oder Mischungen aus Glyzerin und Saccharose eingesetzt, so beträgt der Anteil an Glyzerin in der Mischung ≥ (mindestens) 10 Gew.-%, ≥ 20 Gew.-%, ≥ 40 Gew.-%, ≥ 50 Gew.-%, ≥ 60 Gew.-%, oder ≥ 80 Gew.-% und ≤ (höchstens) 90 Gew.-%.

Werden in einem erfindungsgemäßen Verfahren Mischungen aus Glyzerin und ein Zuckergemisch aus Glukose und Fruktose eingesetzt, so beträgt der Anteil an Glyzerin in der Mischung ≥ (mindestens) 10 Gew.-%, ≥ 20 Gew.-%, ≥ 40 Gew.-%, ≥ 50 Gew.-%, ≥ 60 Gew.-%, oder ≥ 80 Gew.-% und ≤ (höchstens) 90 Gew.-%. Der Anteil an Glukose in dem Zuckergemisch bestehend aus Glukose und Fruktose beträgt ≥ (mindestens) 10 Gew.-%, ≥ 20 Gew.-%, ≥ 40 Gew.-%, ≥ 50 Gew.-%, ≥ 60 Gew.-%, oder ≥ 80 Gew.-% und ≤ (höchstens) 90 Gew.-%, bevorzugt 45 bis 65 Gew.-%, 50 bis 65 Gew.-%, 50 bis 60 Gew.-%, 55 bis 65 Gew.-% oder 55 bis 60 Gew.-%.

Werden in einem erfindungsgemäßen Verfahren Mischungen aus Glyzerin und ein Zuckergemisch aus Saccharose, Glukose und Fruktose eingesetzt, so beträgt der Anteil an Glyzerin in der Mischung ≥ (mindestens) 10 Gew.-%, ≥ 20 Gew.-, ≥ 40 Gew.-%, ≥ 50 Gew.-%, ≥ 60 Gew.-%, oder ≥ 80 Gew.-% und ≤ (höchstens) 90 Gew.-%. Der Anteil an Saccharose, Glukose und Fruktose in dem Zuckergemisch beträgt jeweils > (mehr als) 10 Gew.-% und weniger als 80 Gew.-%.

Wird in einem erfindungsgemäßen Verfahren Glyzerin bzw. Rohglyzerin oder Glyzerin bzw. Rohglyzerin und einer oder mehrere der Zucker ausgewählt aus der Gruppe Glukose, Saccharose und Fruktose als Kohlenstoffquelle eingesetzt, so enthält das Medium im Falle des batch-Verfahrens dann dementsprechend Glyzerin oder Glyzerin und den oder die angegegebenen Zucker.

Im Falle des fed batch-Verfahrens kann die Kohlenstoffquelle auf verschiedenen Arten in das Verfahren eingeführt werden.

In einem ersten Aspekt wird für die batch-Phase einer oder mehrere der angegebenen Zucker vorgelegt. In der sich anschließenden Zulaufphase wird
a) Glyzerin bzw. Rohglyzerin, oder
b) eine Mischung aus Glyzerin und einem oder mehreren der
   angegeben Zucker
   eingesetzt.

In einem zweiten Aspekt wird für die batch-Phase Glyzerin vorgelegt. In der sich anschließenden Zulaufphase wird
a) Glyzerin bzw. Rohglyzerin, oder
b) eine Mischung aus Glyzerin und einem oder mehreren der
   angegeben Zucker
eingesetzt.

In einem dritten Aspekt wird für die batch-Phase eine Mischung aus Glyzerin und einem oder mehreren der angegeben Zucker vorgelegt. In der sich anschließenden Zulaufphase wird
a) Glyzerin bzw. Rohglyzerin, oder
b) eine Mischung aus Glyzerin und einem oder mehreren der
   angegeben Zucker
eingesetzt.

In einem vierten Aspekt wird für die batch-Phase eine Kohlenstoffquelle ausgewählt aus der Gruppe
a) Glyzerin bzw. Rohglyzerin,
b) einer oder mehrere der angegebenen Zucker, und
c) eine Mischung aus Glyzerin und einem oder mehreren der Zucker, bevorzugt ausgewählt aus der Gruppe Saccharose, Glukose und Fruktose
   vorlegt. In der sich anschließenden Zulaufphase wird
d) in mindestens einer zusammenhängenden Periode von mindestens einer (1) Stunde und maximal 100, bevorzugt maximal 50, besonders bevorzugt maximal 30 Stunden Glyzerin und anschließend in mindestens einer zusammenhängenden Periode von mindestens einer (1) Stunde und maximal 100, bevorzugt maximal 50, besonders bevorzugt maximal 30 Stunden einer oder mehrere der angegebenen Zucker eingesetzt, oder
e) in mindestens einer zusammenhängenden Periode von mindestens einer (1) Stunde und maximal 100, bevorzugt maximal 50, besonders bevorzugt maximal 30 Stunden einer oder mehrere der angegebenen Zucker und anschließend in mindestens einer zusammenhängenden Periode von mindestens einer (1) Stunde und maximal 100, bevorzugt maximal 50, besonders bevorzugt maximal 30 Stunden Glyzerin eingesetzt.
wobei der Anteil an Glyzerin an der eingesetzten Kohlenstoffquelle ≥ (mindestens) 10 Gew.-%, ≥ 20 Gew.-%, ≥ 40 Gew.-%, ≥ 50 Gew.-%, ≥ 60 Gew.-%, oder ≥ 80 Gew.-% und ≤ (höchstens) 90 Gew.-% beträgt.

Im Falle eines repeated fed batch-Verfahrens werden für die sich wiederholenden batch- und Zulaufphasen die Kohlenstoffquellen wie für das batch- und fed batch-Verfahren beschrieben (siehe oben) eingesetzt.

Im Falle einer kontinuierlichen Kultur werden Glyzerin oder Glyzerin und einer oder mehrere der Zucker ausgewählt aus der Gruppe Saccharose, Glukose und Fruktose kontinuierlich hinzugefügt.

Als Stickstoffquelle können organische Stickstoff-haltige Stoffgemische wie Peptone, Hefeextrakt, Fleischextrakt, Maisquellwasser oder Sojabohnenmehl, organische Verbindungen wie Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat, Dikaliumhydrogenphosphat oder die entsprechenden Natrium-haltigen Salze einzeln oder als Mischung verwendet werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie zum Beispiel Fettsäurepolyglykolester eingesetzt werden. Zur Aufrechterhaltung der Stabilität von Plasmiden können dem Medium geeignete selektiv wirkende Stoffe zum Beispiel Antibiotika hinzugefügt werden. Um aerobe Bedingungen aufrechtzuerhalten, werden Sauerstoff oder Sauerstoffhaltige Gasmischungen wie zum Beispiel Luft in die Kultur eingetragen. Die Temperatur der Kultur liegt normalerweise bei 25°C bis 45°C und vorzugsweise bei 30°C bis 40°C.

Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Bestandteile des/ der eingesetzten Fermentationsmediums/ Fermentationsmedien bzw. der Einsatzstoffe wie beispielsweise Vitamine wie Thiamin oder Salze wie Magnesiumsulfat.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren zur Bestimmung der Konzentration zu einem oder mehreren Zeitpunkt(en) im Verlauf der Fermentation oder in den verschiedenen Produkten kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC-GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America, USA (1989) 86: 2172-2175) durchgeführt.

Die Bebrütungstemperatur bei der Herstellung von Stämmen und Transformanten ist, wenn nicht anders beschrieben, 37°C.

### Beispiel 1

### Konstruktion des Aminosäure-Austausches (G428D) im glpK-Gen

Teile der stromaufwärts und -abwärts der Positionen 1282-1284 des kodierenden Sequenz des glpK-Gens, entsprechend der Aminosäureposition 428, liegenden Genregionen und ein Teil der 3'-Region des glpK-Gens wurden aus Escherichia coli K12 unter Anwendung der "gene SOEing"-Polymerase-Kettenreaktion (PCR) (Gene Splicing by Overlap Extension, Horton, Molecular Biotechnology 3: 93-98 (1995)) sowie synthetischen Oligonukleotiden amplifiziert. Ausgehend von der Nukleotidsequenz des glpK-Gens und stromabwärts liegender Sequenzen in E. coli K12 MG1655 (SEQ ID No. 7, Accession Number U00096 (Region: 3557870-3558628)) wurden PCR-Primer synthetisiert (MWG Biotech, Ebersberg, Deutschland). Die Primer ermöglichen die Amplifizierung eines ca. 1,5 kb langen DNA-Abschnittes, welcher die glpK(G427D)-Mutation trägt. Die Sequenzen der Primer glpK_4_SOEing und glpK_5_SOEing wurden so modifiziert, dass eine Erkennungsstelle für HaeII entfällt und dafür eine Erkennungsstelle für das Restriktionsenzym BspE1 entsteht, ohne die Aminosäuresequenz des Proteins in diesem Sequenzbereich zu ändern (in der unten dargestellten Nukleotidabfolge durch Unterstreichen markiert):
glpK_3: 5' - CGATTACACCAACGCCTCTC - 3' (SEQ ID No. 9)
glpK_6: 5' - GCCCGACAATCAGCTTCTCC - 3' (SEQ ID No. 12)

Die Primer glpK_4_SOEing und glpK_5_SOEing sind über 27 Nukleotide reverse-komplementär zueinander und tragen in diesem Bereich die Mutation GC -> AT, welche den Aminosäureaustausch G428D bewirkt.

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein 759 bp großes DNA-Fragment aus dem 5'-Bereich der glpK-Mutation (mit glpK1 bezeichnet, PCR-Produkt der Primer glpK_3 und glpK_4_SOEing) und ein 745 bp großes DNA-Fragment aus dem 3'-Bereich der glpK-Mutation (inklusive stromabwärts liegender Sequenzen, mit glpK2 bezeichnet, PCR-Produkt der Primer glpK_6 und glpK_5_SOEing) kann mit den spezifischen Primern unter Standard-PCR-Bedingungen (Innis et al. (1990) PCR Protocols. A Guide to Methods and Applications, Academic Press) mit der Phusion High Fidelity DNA Polymerase (New England Biolabs , Frankfurt, Deutschland) amplifiziert werden. Die beiden PCR-Produkte glpK1 und glpK2 wurden nach einer Kontrolle im 0,8%igen Agarosegel nach herkömmlichen Methoden aufgereinigt (High Pure PCR Product Purification Kit, Roche Diagnostics GmbH, Mannheim, Deutschland) und dann zusammen als Template in eine weitere PCR unter Verwendung der Primer glpK_3 und glpK_6 eingesetzt. Auf diesem Wege erhält man eine glpK-Mutationskassette von 1477 Basenpaaren, welche das 3'-Ende des glpK-Gens mit der Mutation sowie den angrenzenden Bereich aus der stromabwärts liegenden Region des glpK-Gens umfasst und die in SEQ ID No. 13 dargestellte Sequenz aufweist.

Das beschriebene glpK-Allel wurde nach der Auftrennung im 0,8%igen Agarosegel isoliert (QIAGEN QIAquick Gel Extraction Kit, Qiagen GmbH, Hilden, Deutschland) und mit dem Vektor pCR-BluntII-TOPO nach Herstellerangaben (TOPO Cloning Kit Manual, Invitrogen GmbH, Karlsruhe, Deutschland) ligiert.

Der Ligationsansatz wurde in TOP10-Zellen (Invitrogen GmbH, Karlsruhe, Deutschland) transformiert und Plasmid tragende Zellen auf LB Agar (Sambrook et al., Molecular Cloning: A Laboratory Manual. 2nd Ed., Cold Spring Harbor, New York, 1989), der mit 25 µg/ml Kanamycin versetzt ist, bei 37°C selektioniert.

Die erfolgreiche Klonierung wurde nach Plasmid DNA Isolierung (QIAprep Spin Miniprep Kit, Qiagen GmbH, Hilden, Deutschland) durch Spaltung mit dem Enzym HaeII nachgewiesen. Der entstandene Vektor wurde pCRBluntII-glpK(G428D) genannt und mittels Sequenzierung (AGOWA GmbH, Berlin, Deutschland) überprüft.

Danach wurde der Vektor pCRBluntII-glpK(G428D) mit den Restriktionsendonuklease XbaI und SpeI gespalten, durch Elektrophorese in einem 0,8%igen Agarosegel aufgetrennt und das ca. 1,57 kb große glpK(G428D)-Fragment aus dem Gel isoliert, mit den üblichen Methoden aufgereinigt (QIAquick Gel Extraction Kit, Qiagen, Hilden) und mit dem Plasmid pKO3 (Link et al., Journal of Bacteriology 179: 6228-6237 (1997)), das ebenfalls mit dem Enzym XbaI verdaut und mit Alkalischer Phosphatase (Alkaline Phosphatase, Boehringer, Mannheim, Deutschland) behandelt worden ist, mittels Ready-To-Go T4 DNA Ligase (Amersham Pharmacia Biotech, Freiburg, Deutschland) ligiert.

Der Ligationsansatz wurde in DH5α (Grant et al.; Proceedings of the National Academy of Sciences USA, 87 (1990) 4645-4649) transformiert (Hanahan, In. DNA Cloning. A Practical Approach. Vol. 1, ILR-Press, Cold Spring Harbor, New York, 1989) und Plasmid tragende Zellen auf LB Agar (Sambrook et al., Molecular Cloning: A Laboratory Manual. 2nd Ed., Cold Spring Harbor, New York, 1989), der mit 20 µg/ml Chloramphenicol versetzt ist, bei 30°C selektioniert.

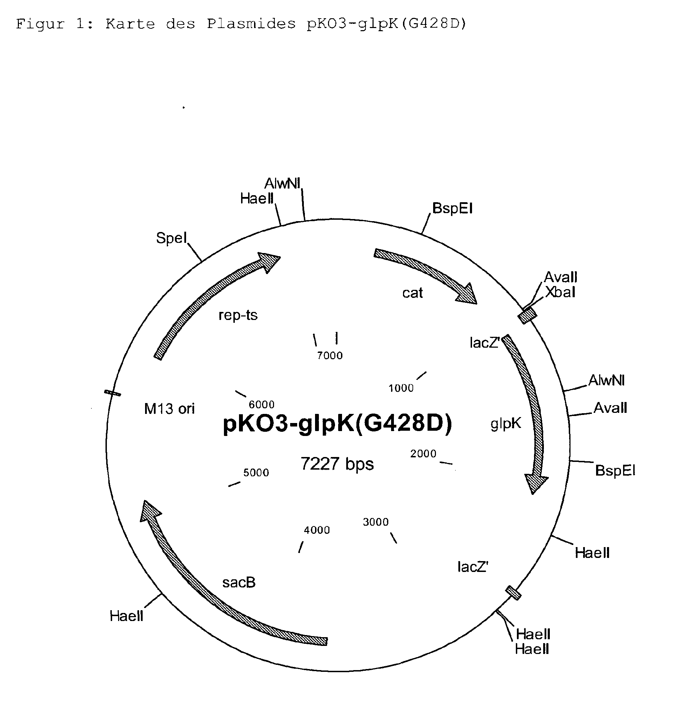

Die erfolgreiche Klonierung wurde nach Plasmid DNA Isolierung (QIAprep Spin Miniprep Kit, Qiagen GmbH, Hilden, Deutschland) und Spaltung mit den Enzym AvaII und AlwNI nachgewiesen. Der entstandene Austauschvektor pKO3-glpK(G428D) ist in Figur 1 dargestellt.

### Beispiel 2

### Konstruktion des Aminosäure-Austausches (G231D) im glpK-Gen

Die stromaufwärts und -abwärts der Positionen 691-693 der kodierenden Sequenz des glpK-Gens, entsprechend der Aminosäureposition 231, liegenden Genregionen wurden aus Escherichia coli K12 unter Anwendung der "gene SOEing"-Polymerase-Kettenreaktion (PCR) (Gene Splicing by Overlap Extension, Horton, Molecular Biotechnology 3: 93-98 (1995)) sowie synthetischen Oligonukleotiden amplifiziert. Ausgehend von der Nukleotidsequenz des glpK-Gens in E. coli K12 MG1655 (SEQ ID No. 7, Accession Number U00096 (Region: 3557870-3558628)) wurden PCR-Primer synthetisiert (MWG Biotech, Ebersberg, Deutschland). Die Primer ermöglichen die Amplifizierung eines ca. 1,55 kb langen DNA-Abschnittes, welcher das glpK(G231D)-Gen bzw. Allel trägt. Die Sequenzen der Primer glpK_7_SOEing und glpK_8_SOEing wurden so modifiziert, dass eine Erkennungsstelle für MluI entfällt und dafür eine Erkennungsstelle für das Restriktionsenzym EcoRI entsteht, ohne die Aminosäuresequenz des Proteins in diesem Sequenzbereich zu ändern (in der unten dargestellten Nukleotidabfolge durch Unterstreichen markiert):
glpK_1 5' - CGGTCGAC ATGACTACGG GACAATTAAAC - 3'
glpFK_2 5' - CGGTCGAC TTATTCGTCG TGTTCTTCCC - 3' (SEQ ID No. 17)

Die Primer glpK_7_SOEing und glpK_8_SOEing sind über 29 Nukleotide reverse-komplementär zueinander und tragen in diesem Bereich die Mutation GC -> AT, welche den Aminosäureaustausch G231D bewirkt.

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein 744 bp großes DNA-Fragment aus dem 5'-Bereich der glpK-Mutation (mit glpK5 bezeichnet, PCR-Produkt der Primer glpK_1 und glpK_7_SOEing) und ein 827 bp großes DNA-Fragment aus dem 3'-Bereich der glpK-Mutation (mit glpK6 bezeichnet, PCR-Produkt der Primer glpFK_2 und glpK_8_SOEing) kann mit den spezifischen Primern unter Standard-PCR-Bedingungen (Innis et al. (1990) PCR Protocols. A Guide to Methods and Applications, Academic Press) mit der Phusion High Fidelity DNA Polymerase (New England Biolabs , Frankfurt, Deutschland) amplifiziert werden. Die beiden PCR-Produkte glpK5 und glpK6 wurden nach einer Kontrolle im 0,8%igen Agarosegel nach herkömmlichen Methoden aufgereinigt (High Pure PCR Product Purification Kit, Roche Diagnostics GmbH, Mannheim, Deutschland) und dann zusammen als Template in eine weitere PCR unter Verwendung der Primer glpK_1 und glpFK_2 eingesetzt. Auf diesem Wege erhält man eine glpK-Mutationskassette von 1546 Basenpaaren, welche das glpK-Gen mit der Mutation G231D umfasst und die in SEQ ID No. 18 dargestellte Sequenz aufweist.

Das beschriebene glpK-Allel wurde nach der Auftrennung im 0,8%igen Agarosegel isoliert (QIAGEN QIAquick Gel Extraction Kit, Qiagen GmbH, Hilden, Deutschland) und mit dem Vektor pCR-BluntII-TOPO nach Herstellerangaben (TOPO Cloning Kit Manual, Invitrogen GmbH, Karlsruhe, Deutschland) ligiert.

Der Ligationsansatz wurde in TOP10-Zellen (Invitrogen GmbH, Karlsruhe, Deutschland) transformiert und Plasmid tragende Zellen auf LB Agar (Sambrook et al., Molecular Cloning: A Laboratory Manual. 2nd Ed., Cold Spring Harbor, New York, 1989), der mit 25 µg/ml Kanamycin versetzt ist, bei 37°C selektioniert.

Die erfolgreiche Klonierung wurde nach Plasmid DNA Isolierung (QIAprep Spin Miniprep Kit, Qiagen GmbH, Hilden, Deutschland) durch Spaltung mit dem Enzym MluI nachgewiesen. Der entstandene Vektor wird pCRBluntII-glpK(G231D) genannt und mittels Sequenzierung (AGOWA GmbH, Berlin, Deutschland) überprüft.

Danach wurde der Vektor pCRBluntII-glpK(G231D) mit der Restriktionsendonuklease SalI gespalten, durch Elektrophorese in einem 0,8%igen Agarosegel aufgetrennt und das 1536 bp große glpK(G231D)-Fragment aus dem Gel isoliert, mit den üblichen Methoden aufgereinigt (QIAquick Gel Extraction Kit, Qiagen, Hilden) und mit dem Plasmid pKO3 (Link et al., Journal of Bacteriology 179: 6228-6237 (1997)), das ebenfalls mit dem Enzym SalI verdaut und mit Alkalischer Phosphatase (Alkaline Phosphatase, Boehringer, Mannheim, Deutschland) behandelt worden ist, mittels Ready-To-Go T4 DNA Ligase (Amersham Pharmacia Biotech, Freiburg, Deutschland) ligiert.

Der Ligationsansatz wurde in DH5α (Grant et al.; Proceedings of the National Academy of Sciences USA, 87 (1990) 4645-4649) transformiert (Hanahan, In. DNA Cloning. A Practical Approach. Vol. 1, ILR-Press, Cold Spring Harbor, New York, 1989) und Plasmid tragende Zellen auf LB Agar (Sambrook et al., Molecular Cloning: A Laboratory Manual. 2nd Ed., Cold Spring Harbor, New York, 1989), der mit 20 µg/ml Chloramphenicol versetzt ist, bei 30°C selektioniert.

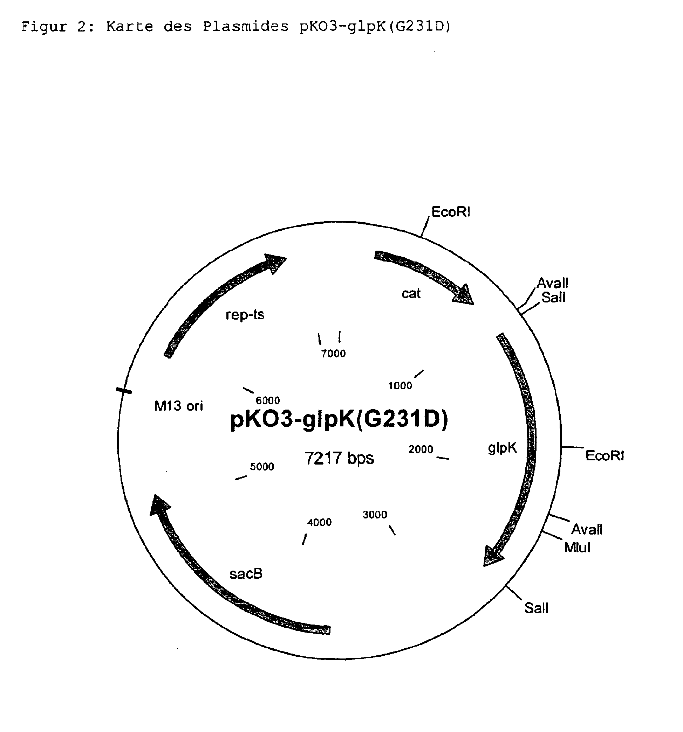

Die erfolgreiche Klonierung wurde nach Plasmid DNA Isolierung (QIAprep Spin Miniprep Kit, Qiagen GmbH, Hilden, Deutschland) und Spaltung mit den Enzym EcoRI und AvaII nachgewiesen. Der entstandene Austauschvektor pKO3-glpK(G231D) ist in Figur 2 dargestellt.

### Beispiel 3

### Ortsspezifische Mutagenese des glpK-Gens in dem E. coli Stamm MG442

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 und bei der Deutschen Sammlung für Mikroorganismen und Zellkulturen (DSMZ, Braunschweig, Deutschland) in Übereinstimmung mit dem Budapester Vertrag als DSM 16574 hinterlegt.

Für den Austausch des chromosomalen glpK-Gens gegen die Plasmid-kodierten Mutationskonstrukte wurde MG442 mit den Plasmiden pKO3-glpK(G428D) und pKO3-glpK(G231D) transformiert. Der Genaustausch erfolgt mit dem von Link et al. (Journal of Bacteriology 179: 6228-6237 (1997)) beschriebenen Selektionsverfahren und wurde durch Standard-PCR-Methoden (Innis et al. (1990) PCR Protocols. A Guide to Methods and Applications, Academic Press) mit folgenden Oligonukleotid Primern und anschließende Restriktion mit BspE1 bzw. SpeI verifiziert:
Für glpK(G428D):
   glpK_3: 5' - CGATTACACCAACGCCTCTC - 3' (SEQ ID No. 9)
   glpK_6: 5' - GCCCGACAATCAGCTTCTCC - 3' (SEQ ID No. 12)
Für glpK (G231D) :

Nach erfolgtem Austausch liegen in MG442 die in SEQ ID No. 20 dargestellte Form des glpK-Allels (G428D) bzw. die in SEQ ID No. 19 dargestellte Form des glpK-Allels (G231D) (Sequenzierung durch AGOWA GmbH, Berlin, Deutschland) vor. Die erhaltenen Stämme werden als MG442glpK(G428D) bzw. MG442glpK(G231D) bezeichnet.

### Beispiel 4

### Herstellung von L-Threonin mit den Stämmen MG442glpK(G428D) und MG442glpK(G231D)

Die Stämme MG442glpK(G428D), MG442glpK(G231D) und MG442 wurden auf Minimalmedium mit der folgenden Zusammensetzung vermehrt: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar. Die Bildung von L-Threonin wurde in batch Kulturen von 10 ml, die in 100 ml Erlenmeierkolben enthalten sind, überprüft. Dazu wird 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glycerin beimpft und für 16 Stunden bei 37°C und 180 rpm auf einem ESR Inkubator der Firma Kühner AG (Birsfelden, Schweiz) inkubiert. 250 µl dieser Vorkultur werden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glycerin) überimpft und für 48 Stunden bei 37°C inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

Anschließend wurde die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442 | 6,0 | 1,8 |
| MG442glpK(G428D) | 5, 9 | 2,8 |
| MG442glpK(G231D) | 6,3 | 2,7 |

### Beispiel 5

### Ortsspezifische Mutagenese des glpK-Gens in dem E. coli Stamm DM1831/pMU91

Der L-Tryptophan produzierende E. coli Stamm Stamm DM1831/pMU91 ist ein durch P1-Transduktion erhältliches trpS⁺ Derivat des in der Patentschrift US-A-5,756,345 beschriebenen Escherichia coli K-12 Stammes JP6015/pMU91. pMU91 ist ein Plasmid abgeleitet von pSC101 (Cohen et al., Journal of Bacteriology 132: 734-737 (1977)), welches Tet^{R}, trpE476DCBA und serA⁺ trägt. JP6015/pMU91 ist hinterlegt als DSM 10123 bei der Deutschen Sammlung für Mikroorganismen und Zellkulturen (DSMZ, Braunschweig, Deutschland) in Übereinstimmung mit dem Budapester Vertrag.

Nach Vermehrung in Antibiotika-freiem LB-Medium für circa sechs Generationen wurde ein Derivat des Stammes DM1831/pMU91 isoliert, welches das Plasmid pMU91 nicht mehr trägt. Der erhaltene Stamm ist Tetracyclin-sensitiv und wird als DM1831 bezeichnet.

Für den Austausch des chromosomalen glpK-Gens gegen die Plasmid-kodierten Mutationskonstrukte wurde DM1831 mit den Plasmiden pKO3-glpK(G428D) und pKO3-glpK(G231D) transformiert. Der Genaustausch erfolgt mit dem von Link et al. (Journal of Bacteriology 179: 6228-6237 (1997)) beschriebenen Selektionsverfahren und wurde durch Standard-PCR-Methoden (Innis et al. (1990) PCR Protocols. A Guide to Methods and Applications, Academic Press) mit folgenden Oligonukleotid Primern und anschließende Restriktion mit BspE1 bzw. SpeI verifiziert:
Für glpK(G428D):
   glpK_3: 5' - CGATTACACCAACGCCTCTC - 3' (SEQ ID No. 9) glpK_6: 5' - GCCCGACAATCAGCTTCTCC - 3' (SEQ ID No. 12)
Für glpK(G231D) :
   glpK_1: 5' - CGGTCGAC ATGACTACGG GACAATTAAAC - 3'
   (SEQ ID No. 14) glpFK_2: 5' - CGGTCGAC TTATTCGTCG TGTTCTTCCC - 3' (SEQ ID No. 17)

Nach erfolgtem Austausch liegen in DM1831 die in SEQ ID No. 20 dargestellte Form des glpK-Allels (G428D) bzw. die in SEQ ID No. 19 dargestellte Form des glpK-Allels (G231D) (Sequenzierung durch AGOWA GmbH, Berlin, Deutschland) vor. Die erhaltenen Stämme werden als DM1831glpK(G428D) bzw. DM1831glpK(G231D) bezeichnet.

Das in der Patentschrift US-A-5,756,345 beschriebene Plasmid pMU91, welches die genetischen Informationen für die Tryptophan-Produktion trägt, wurde aus dem Stamm JP6015/pMU91 isoliert. Das Plasmid ist in Figur 3 dargestellt. Die Stämme DM1831glpK(G428D) und DM1831glpK(G231D) wurden mit diesem Plasmid transformiert. Je einer der erhaltenen Transformanten wird als DM1831glpK(G428D)/pMU91 bzw. DM1831glpK(G231D)/pMU91 bezeichnet.

### Beispiel 6

### Herstellung von L-Tryptophan mit den Stämmen DM1831glpK(G428D)/pMU91 und DM1831glpK(G231D)/pMU91

Die Stämme DM1831glpK(G428D)/pMU91, DM1831glpK(G231D)/pMU91 und DM1831/pMU91 wurden auf LB-Medium mit der folgenden Zusammensetzung: 10 g/l Bacto-Trypton, 5 g/l Hefe-Extrakt, 10 g/l NaCl (pH-Einstellung auf 7,5 mit NaOH), 2 g/l Glucose, 20 g/l Agar und 5 mg/l Tetracyclin bei 30°C weiter vermehrt. Die Bildung von L-Tryptophan wurde in batch Kulturen von 10 ml, die in 100 ml Erlenmeierkolben enthalten sind, überprüft. Dazu wird 10 ml Vorkulturmedium der folgenden Zusammensetzung: 1 g/l Hefeextrakt, 100 ml/l MOPS Puffer (10x), 10 g/l Glycerin und 5 mg/l Tetracyclin beimpft und für 16 Stunden bei 30°C und 150 rpm auf einem ESR Inkubator der Firma Kühner AG (Birsfelden, Schweiz) inkubiert.

10xMOPS Puffer wird aus den Lösungen A und B entsprechend den Tabellen 2 bis 4 hergestellt, zwei Volumen von Lösung A werden steril zu drei Volumen der Lösung B gegeben.

**Tabelle 2: Lösung A für 10xMOPS Puffer (sterilfiltriert).**

| Komponente | Konzentration |
|---|---|
| MOPS (Morpholinopropansulfonsäure) | 419 g/L |
| KOH (fest) | Zur Einstellung des pH-Wertes auf 7,4 |

**Tabelle 3: Lösung B für 10xMOPS Puffer. Sterilisiert durch Autoklavieren (30 Minuten, 121°C).**

| Komponente | Konzentration |
|---|---|
| Na₃ Citrat x 2H₂O | 2,35 g/l |
| FeSO₄ x 7H₂O | 0,22 g/l |
| NH₄Cl | 32,0 g/l |
| MgSO₄ x 7H₂O | 6,7 g/l |
| KCl | 4, 0 g/l |
| CaCl₂ x 2H₂O | 0,25 mg/l |
| Stammlösung Spurenelemente (Tab. 4) | 3,33 ml/l |

**Tabelle 4: Stammlösung der Spurenelemente (in demin. Wasser) für 10xMOPS Puffer.**

| Komponente | Konzentration |
|---|---|
| (NH₄) ₆Mo₇O₂₄ x 4H₂O | 3,7 mg/l |
| H₃BO₃ | 24,0 mg/l |
| CoCl₂ x 6H₂O | 7,1 mg/l |
| ZnSO₄ x 7H₂O | 28,7 mg/l |
| MnCl₂ x 4H₂O | 15,8 mg/l |
| CuSO₄ x 5H₂O | 2,5 mg/l |

Je 250 µl dieser Vorkultur werden in 10 ml Produktionsmedium PM1 entsprechend Tabelle 5 überimpft und für 72 Stunden bei 30°C und 150 rpm inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

**Tabelle 5: PM1 Medium: für Produktionstests in 100 ml Erlenmeierkolben**

| Komponente | Konzentration |
|---|---|
| 10xMOPS Puffer | 100 ml/l |
| Thiamine HCl (0,01%) | 1,0 ml/l |
| KH2PO4 (15mM) | 10 ml/l |
| Glycerin | 10 g/l |
| Tetracyclin | 5 mg/l |

Anschließend wurde die Konzentration an gebildetem L-Tryptophan im steril filtrierten Kulturüberstand durch reversed phase HPLC, so wie bei Lindroth et al. (Analytical Chemistry (1979) 51: 1167-1174) beschrieben, bestimmt.

In Tabelle 6 ist das Ergebnis des Versuches dargestellt.

**Tabelle 6**

| Stamm | OD (660 nm) | L-Tryptophan g/l |
|---|---|---|
| DM1831/pMU91 | 1,5 | 0,85 |
| DM1831glpK(G428D)/pMU91 | 1,6 | 1,2 |
| DM1831glpK(G231D)/pMU91 | 1,7 | 1,0 |

### Beispiel 7

### Konstruktion des Expressionsplasmides pMW218glpK

Das Gen glpK aus E. coli K12 wurde unter Anwendung der Polymerase-Kettenreaktion (PCR) sowie synthetischer Oligonukleotiden amplifiziert. Ausgehend von der Nukleotidsequenz des glpK-Gens in E. coli K12 MG1655 (Accession Number U00096 (Region: 4113737-4115245)) wurden PCR-Primer synthetisiert (MWG Biotech, Ebersberg, Deutschland). Die Sequenzen der Primer wurden so modifiziert, dass Erkennungsstellen für Restriktionsenzyme entstanden. Für beide Primer wurde die Erkennungssequenz für SalI gewählt, die in der unten dargestellten Nukleotidabfolge durch Unterstreichen markiert ist:
glpK_1 5' - CGGTCGAC ATGACTACGG GACAATTAAAC - 3' (SEQ ID No. 14)
glpFK 2 5' - CGGTCGAC TTATTCGTCG TGTTCTTCCC - 3' (SEQ ID No. 17)

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein 1546 bp großes DNA-Fragment konnte mit den spezifischen Primern unter Standard-PCR-Bedingungen (Innis et al. (1990) PCR Protocols. A Guide to Methods and Applications, Academic Press) mit der Phusion High-Fidelity Polymerase (New England Biolabs, Frankfurt, Deutschland) amplifiziert werden.

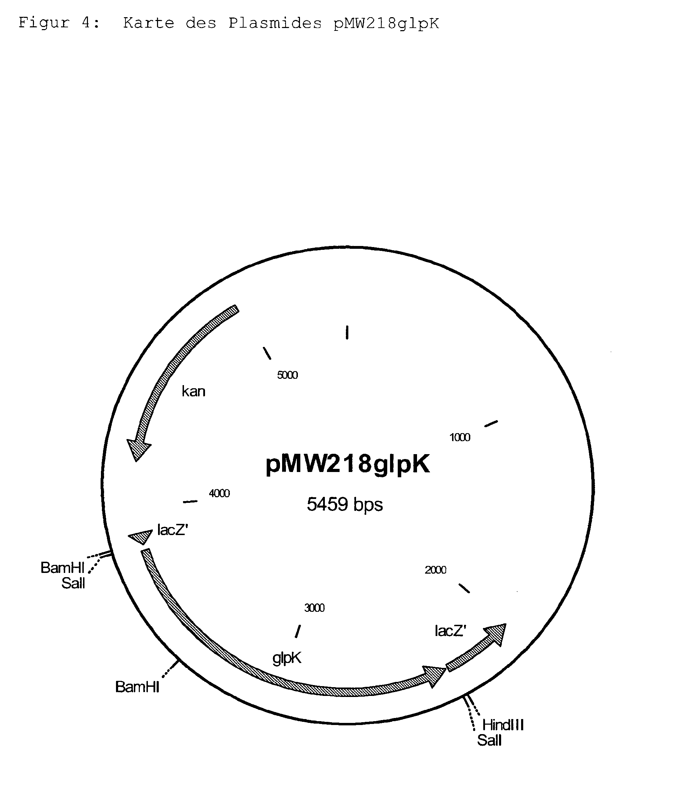

Das PCR-Produkt (die Aminosäuresequenz des korrespondierenden Proteins in entspricht der in SEQ ID No. 2) wurde mit dem Restriktionsenzym SalI gespalten und mit dem Vektor pMW218 (Nippon Gene, Toyama, Japan), der ebenfalls mit dem Enzym SalI verdaut worden ist, ligiert. Der E. coli Stamm TOP10 (Invitrogen, Groningen, Niederlande) wurde mit dem Ligationsansatz transformiert und Plasmid tragende Zellen auf LB Agar, der mit 50 µg/ml Kanamycin versetzt ist, selektioniert. Die erfolgreiche Klonierung konnte nach der Plasmid DNA Isolierung durch die Kontrollspaltung mit den Enzymen BamHI und SalI nachgewiesen werden. Das Plasmid wird als pMW218glpK (Figur 4) bezeichnet.

### Beispiel 8

### Konstruktion des Expressionsplasmides pMW219glpK(G428D)

Das Gen glpK aus E. coli K12 wurde unter Anwendung der Polymerase-Kettenreaktion (PCR) sowie synthetischer Oligonukleotiden so amplifiziert, dass es die glpK(G427D)-Mutation trägt. Ausgehend von der Nukleotidsequenz des glpK-Gens in E. coli K12 MG1655 (Accession Number U00096 (Region: 4113737-4115245)) wurden PCR-Primer synthetisiert (MWG Biotech, Ebersberg, Deutschland):

Die Sequenzen der Primer glpK_1 und glpFK_2 wurden so modifiziert, dass Erkennungsstellen für das Restriktionsenzym SalI entstanden. Die Sequenzen der Primer glpK_4_SOEing und glpK_5_SOEing wurden so modifiziert, dass eine Erkennungsstelle für HaeII entfällt und dafür eine Erkennungsstelle für das Restriktionsenzym BspE1 entsteht (in der unten dargestellten Nukleotidabfolge durch Unterstreichen markiert):
glpK_1 5' - CGGTCGAC ATGACTACGG GACAATTAAAC - 3' (SEQ ID No. 14)
glpFK_2 5' - CGGTCGAC TTATTCGTCG TGTTCTTCCC - 3' (SEQ ID No. 17)

Die Primer glpK_4_SOEing und glpK_5_SOEing sind über 27 Nukleotide reverse-komplementär zueinander und tragen in diesem Bereich die Mutation GC -> AT, welche den Aminosäureaustausch G428D bewirkt.

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein 1333 bp großes DNA-Fragment aus dem 5'-Bereich der glpK-Mutation (mit glpK3 bezeichnet, PCR-Produkt der Primer glpK_1 und glpK_4_SOEing) und ein 240 bp großes DNA-Fragment aus dem 3'-Bereich der glpK-Mutation (mit glpK4 bezeichnet, PCR-Produkt der Primer glpFK_2 und glpK_5_SOEing) kann mit den spezifischen Primern· unter Standard-PCR-Bedingungen (Innis et al. (1990) PCR Protocols. A Guide to Methods and Applications, Academic Press) mit der Phusion High Fidelity DNA Polymerase (New England Biolabs , Frankfurt, Deutschland) amplifiziert werden. Die beiden PCR-Produkte glpK3 und glpK4 wurden nach einer Kontrolle im 0,8%igen Agarosegel nach herkömmlichen Methoden aufgereinigt (High Pure PCR Product Purification Kit, Roche Diagnostics GmbH, Mannheim, Deutschland) und dann zusammen als Template in eine weitere PCR unter Verwendung der Primer glpK_1 und glpFK_2 eingesetzt. Auf diesem Wege erhält man ein glpK-Allel von 1546 Basenpaaren.

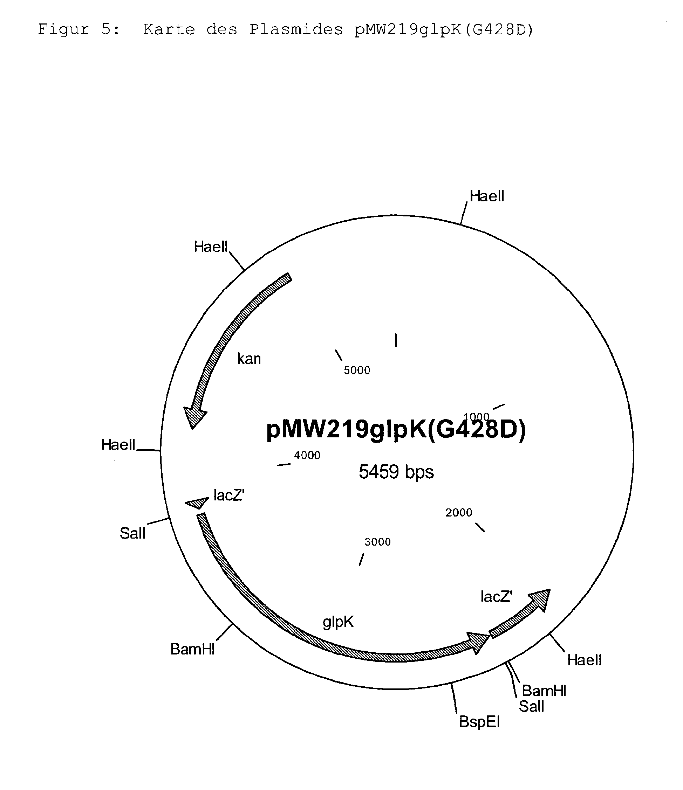

Das PCR-Produkt (die Aminosäuresequenz des korrespondierenden Proteins entspricht der in SEQ ID No. 20) wurde mit dem Restriktionsenzym SalI gespalten und mit dem Vektor pMW219 (Nippon Gene, Toyama, Japan), der ebenfalls mit dem Enzym SalI verdaut worden ist, ligiert. Der E. coli Stamm TOP10 (Invitrogen, Groningen, Niederlande) wurde mit dem Ligationsansatz transformiert und Plasmid tragende Zellen auf LB Agar, der mit 50 µg/ml Kanamycin versetzt ist, selektioniert. Die erfolgreiche Klonierung konnte nach der Plasmid DNA Isolierung durch die Kontrollspaltung mit den Enzymen BamHI und SalI nachgewiesen werden. Das Plasmid wird als pMW219glpK(G428D) (Figur 5) bezeichnet.

### Beispiel 9

### Konstruktion des Expressionsplasmides pMW219glpK(G231D)

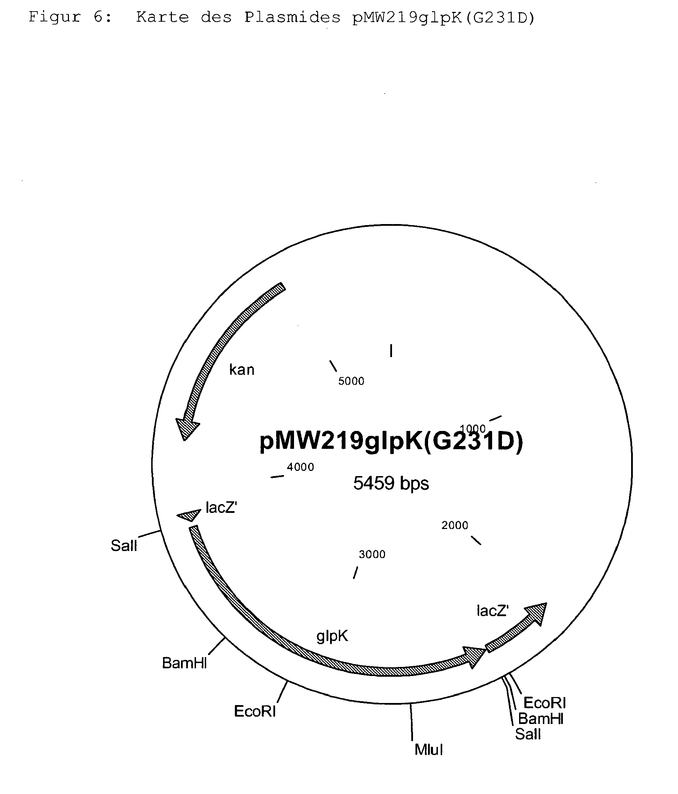

Das ca. 1,54 kb große SalI-geschnittene glpK(G231D)-Fragment (die Sequenz ist dargestellt in SEQ ID No. 18, die Aminosäuresequenz des korrespondierenden Proteins in SEQ ID No. 19) aus Beispiel 2 wurde mit dem Vektor pMW219 (Nippon Gene, Toyama, Japan), der ebenfalls mit dem Enzym SalI verdaut worden ist, ligiert. Der E. coli Stamm TOP10 (Invitrogen, Groningen, Niederlande) wurde mit dem Ligationsansatz transformiert und Plasmid tragende Zellen auf LB Agar, der mit 50 µg/ml Kanamycin versetzt ist, selektioniert. Die erfolgreiche Klonierung konnte nach der Plasmid DNA Isolierung durch die Kontrollspaltung mit den Enzymen BamHI und SalI nachgewiesen werden. Das Plasmid wird als pMW219glpK(G231D) (Figur 6) bezeichnet.

### Beispiel 10

Herstellung von L-Threonin mit den Stämmen MG442/pMW218glpK, MG442/pMW219glpK(G428D) und MG442/pMW219glpK(G231D)

Der Stamm MG442 wurde mit den in den Beispielen 7, 8 und 9 beschriebenen Expressionsplasmiden pMW218glpK, pMW219glpK(G428D), pMW219glpK(G231D) und mit den Vektoren pMW218 und pMW219 (unterscheiden sich nur in der Orientierung der Multiple Cloning Site) transformiert und auf LB Agar mit 50 µg/ml Kanamycin auf Plasmid tragende Zellen selektioniert. Die erfolgreiche Transformation konnte nach der Plasmid DNA Isolierung durch die Kontrollspaltung mit den Enzymen BamHI und SalI nachgewiesen werden. Auf diese Weise entstehen die Stämme MG442/pMW218glpK, MG442/pMW219glpK(G428D), MG442/pMW219glpK(G231D), MG442/pMW219 und MG442/pMW218. Ausgewählte Einzelkolonien wurden anschließend auf Minimalmedium mit der folgenden Zusammensetzung: 3,5 g/l Na₂HPO₄*2H₂O, 1,5 g/l KH₂PO₄, 1 g/l NH₄Cl, 0,1 g/l M₉SO₄*7H₂O, 2 g/l Glucose, 20 g/l Agar und 50 mg/l Kanamycin weiter vermehrt. Die Bildung von L-Threonin wurde wie in Beispiel 4 beschrieben bestimmt.

In Tabelle 7 ist das Ergebnis des Versuches dargestellt.

**Tabelle 7**

| Stamm | OD (660 nm) | L-Threonin g/l |
|---|---|---|
| MG442/pMW218 | 6,0 | 1,8 |
| MG442/pMW219 | 5,9 | 1,8 |
| MG442/pMW218glpK | 4,6 | 2,0 |
| MG442/pMW219glpK(G428D) | 5,8 | 3,1 |
| MG442/pMW219glpK(G231D) | 6,1 | 2,8 |

### Beispiel 11

### Herstellung von L-Tryptophan mit den Stämmen DM1831/pMU91/pMW218glpK, DM1831/pMU91/pMW219glpK(G428D) und DM1831/pMU91/pMW219glpK(G231D)

Der Stamm DM1831/pMU91 wurde mit den in den Beispielen 7, 8 und 9 beschriebenen Expressionsplasmiden pMW218glpK, pMW219glpK(G428D), pMW219glpK(G231D) und mit den Vektoren pMW218 und pMW219 (unterscheiden sich nur in der Orientierung der Multiple Cloning Site) transformiert und auf LB Agar mit 50 µg/ml Kanamycin und 5µg/ml Tetracyclin auf Plasmid tragende Zellen selektioniert. Die erfolgreiche Transformation konnte nach der Plasmid DNA Isolierung durch die Kontrollspaltung mit dem Enzym HindIII nachgewiesen werden. Auf diese Weise entstehen die Stämme DM1831/pMU91/pMW218glpK, DM1831/pMU91/pMW219glpK(G428D), DM1831/pMU91/pMW219glpK(G231D), DM1831/pMU91/pMW218 und DM1831/pMU91/pMW219. Ausgewählte Einzelkolonien wurden anschließend auf LB-Medium mit der folgenden Zusammensetzung: 10 g/l Bacto-Trypton, 5 g/l Hefe-Extrakt, 10 g/l NaCl (pH-Einstellung auf 7,5 mit NaOH), 2 g/l Glucose, 20 g/l Agar, 5 mg/l Tetracyclin und 50 mg/l Kanamycin bei 30°C weiter vermehrt. Die Bildung von L-Tryptophan wurde wie in Beispiel 6 beschrieben bestimmt.

In Tabelle 8 ist das Ergebnis des Versuches dargestellt.

**Tabelle 8**

| Stamm | OD (660 nm) | L-Tryptophan g/l |
|---|---|---|
| DM1831/pMU91/pMW218 | 1,2 | 0,7 |
| DM1831/pMU91/pMW219 | 1,1 | 0,7 |
| DM1831/pMU91/pMW218glpK | 1,1 | 0,9 |
| DM1831/pMU91/pMW219glpK (G428D) | 1,0 | 1,1 |
| DM1831/pMU91/pMW219glpK (G231D) | 1,3 | 1,1 |

Kurze Beschreibung der Figuren:
- Figur 1: Karte des Plasmides pKO3-glpK(G428D)
- Figur 2: Karte des Plasmides pKO3-glpK(G231D)

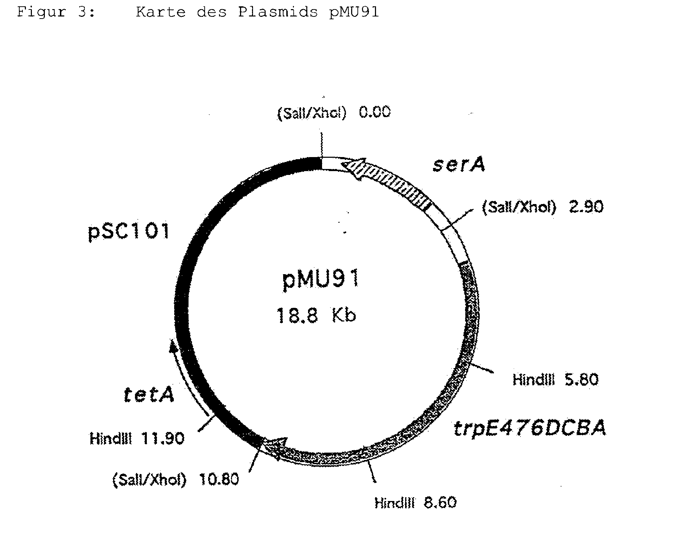

- Figur 3: Karte des Plasmides pMU91
- Figur 4: Karte des das glpK-Gen enthaltenden Plasmides pMW218glpK
- Figur 5: Karte des das glpK-Gen enthaltenden Plasmides pMW219glpK(G428D)
- Figur 6: Karte des das glpK-Gen enthaltenden Plasmides pMW219glpK(G231D)

Längenangaben sind als ca.-Angaben aufzufassen. Die verwendeten Abkürzungen und Bezeichnungen haben folgende Bedeutung:
- cat: Chloramphenicolresistenzgen
- M13 ori: Replikationsursprung
- rep-ts: temperatursensitive Replikationsregion des Plasmides pSC101
- glpK1: Teil der 5'-Region des glpK-Gens
- glpK2: Teil der 3'-Region des glpK-Gens und der stromabwärts liegenden Region
- sacB: sacB-Gen
- pSC101: Plasmidfragment pSC101
- serA: Kodierregion des serA-Gens kodierend für die D-3-Phosphoglycerat-Dehydrogenase
- trpE476DCBA: Teil des Tryptophan-Operons trpLEDCBA mit trpE476-Allel
- tetA : Tetracyclinresistenzgen
- kan: Gen, welches für die Kanamycinresistenz kodiert
- glpK: Kodierregion des glpK-Gens
- lacZ': Genfragment, welches für das α-Peptid der β-Galaktosidase kodiert

Die Abkürzungen für die Restriktionsenzyme haben folgende Bedeutung
- HindIII: Restriktionsendonuklease aus Haemophilus influenzae
- AvaII: Restriktionsendonuklease aus Anabaena variabilis
- AlwNI: Restriktionsendonuklease aus Acinetobacter lwoffii
- BspE1: Restriktionsendonuklease aus einer Bacillus Spezies
- SalI: Restriktionsendonuklease aus Streptomyces albus
- XhoI: Restriktionsendonuklease aus Xanthomonas holcicola
- BamHI: Restriktionsendonuklease aus Bacillus amyloliquefaciens H
- HaeII: Restriktionsendonuklease aus Haemophilus aegyptius
- SpeI: Restriktionsendonuklease aus einer Sphaerotilus Spezies
- MluI: Restriktionsendonuklease aus Micrococcus luteus

## Patentansprüche

1. Verfahren zur Herstellung von L-Aminosäuren, **dadurch gekennzeichnet, dass** man folgende Schritte durchführt:
1.1 Fermentation von L-Aminosäure ausscheidenden rekombinanten Mikroorganismen der Familie Enterobacteriaceae oder der coryneformen Bakterien, welche ein glpK-Gen enthalten, das für ein Polypeptid mit Glyzerinkinase-Aktivität (ATPabhängig) kodiert, dessen Aminosäuresequenz zu mindestens 95% identisch ist mit der von SEQ ID NO: 2, 4 oder 6, wobei das Polypeptid eine Länge von 502 Aminosäuren besitzt, wobei das Polypeptid an Position 428 jede proteinogene Aminosäure, ausgenommen Glycin, enthält, in einem Medium bei einer gewünschten Temperatur, wobei als Kohlenstoffquelle im Wesentlichen Glyzerin oder im Wesentlichen Glyzerin und einer oder mehrere der Zucker ausgewählt aus der Gruppe Glukose, Saccharose und Fruktose eingesetzt wird, und
1.2 Akkumulation der entstehenden L-Aminosäure in der Fermentationsbrühe.

2. Verfahren gemäß den Anspruch 1, **dadurch gekennzeichnet, dass**
die proteinogene Aminosäure an Position 428 L-Glutaminsäure, L-Asparaginsäure, L-Isoleucin, L-Histidin, L-Tryptophan oder L-Phenylalanin ist.

3. Verfahren gemäß Anspruch 2, **dadurch gekennzeichnet, dass** es sich bei der proteinogenen Aminosäure
an Position 428 um L-Asparaginsäure handelt.

4. Verfahren gemäß den Ansprüchen 1 bis 3, **dadurch gekennzeichnet, dass** das kodierte Polypeptid die Aminosäuresequenz von SEQ ID NO: 2, SEQ ID NO: 4 oder SEQ ID NO: 6 einschließlich des genannten Aminosäureaustauschs an Position 428 umfasst, wobei die Aminosäuresequenz zusätzlich eines oder mehrere der Merkmale, ausgewählt aus der Gruppe ein oder höchstens 10 konservative Aminosäureaustausche, eine Verlängerung am C-Terminus um eine bis höchstens 20 Aminosäure(n) .

5. Verfahren gemäß den Ansprüchen 1 bis 4, **dadurch gekennzeichnet, dass** das kodierte Polypeptid die Aminosäuresequenz von SEQ ID NO: 2, SEQ ID NO: 4 oder SEQ ID NO: 6 einschließlich des genannten Aminosäureaustauschs an Position 428 umfasst.

6. Verfahren gemäß Anspruch 4 oder 5, **dadurch gekennzeichnet, dass** das kodierte Polypeptid die Aminosäuresequenz von SEQ ID NO: 2 einschließlich des genannten Aminosäureaustauschs an Position 428 umfasst.

7. Verfahren gemäss den Ansprüchen 1 bis 6, **dadurch gekennzeichnet, dass** man zur Herstellung der L-Aminosäuren Mikroorganismen der Familie Enterobacteriaceae fermentiert, in denen man zusätzlich gleichzeitig das für den Repressor des glp-Regulons kodierende glpR-Gen abschwächt, insbesondere ausschaltet oder die Expression verringert.

8. Verfahren gemäß den Ansprüchen 1 bis 7, **dadurch gekennzeichnet, dass** man zur Herstellung der L-Aminosäuren Mikroorganismen der Familie Enterobacteriaceae fermentiert, in denen man zusätzlich gleichzeitig eines oder mehrere der Gene, ausgewählt aus der Gruppe:
a) das für die große Untereinheit der sn-Glyzerin-3-Phosphat-Dehydrogenase (anaerob) kodierende glpA-Gen,
b) das für die Untereinheit zur Membranverankerung der sn-Glyzerin-3-Phosphat-Dehydrogenase (anaerob) kodierende glpB-Gen,
c) das für die kleine Untereinheit der sn-Glyzerin-3-Phosphat-Dehydrogenase (anaerob) kodierende glpC-Gen,
d) das für die Glyzerin-3-Phosphat-Dehydrogenase (aerob) kodierende glpD-Gen,
e) das für die Schwefeltransferase kodierende glpE-Gen,
f) das für den Glyzerinfascilitator GlpF kodierende glpF-Gen,
g) das glpG-Gen
h) das für die periplasmatische
Glyzerinphosphodiesterase kodierende glpQ-Gen,
i) das für die Glyzerin-3-Phosphat-Permease kodierende glpT-Gen,
j) das für die Fruktose-1,6-Bisphosphatase II kodierende glpX-Gen,
k) das für die Triosephosphat-Isomerase kodierende tpiA-Gen,
l) das für die Glyzerin-Dehydrogenase (NAD-abhängig) kodierende gldA-Gen,
m) das für die N-terminale Domäne der Dihydroxyaceton-Kinase kodierende dhaK-Gen,
n) das für die C-terminale Domäne der Dihydroxyaceton-Kinase kodierende dhaL-Gen,
o) das für die PTS-Protein-Untereinheit der Dihydroxyaceton-Kinase kodierende dhaM-Gen,
p) das für den Aktivator des dha-Operons kodierende dhaR-Gen,
q) das für die Fruktose-6-Phosphat-Aldolase I kodierende fsa-Gen, und
r) das für die Fruktose-6-Phosphat-Aldolase II
kodierende talC-Gen
verstärkt, insbesondere überexprimiert.

9. Verfahren gemäß den Ansprüchen 1 bis 8, **dadurch gekennzeichnet, dass** es sich bei den Mikroorganismen der Familie Enterobacteriaceae um einen Mikroorganismus ausgewählt aus den Gattungen Escherichia, Erwinia, Providencia und Serratia handelt.

10. Verfahren gemäß den Ansprüchen 1 bis 9, **dadurch gekennzeichnet, dass** als Kohlenstoffquelle im Wesentlichen Glyzerin eingesetzt wird.

11. Verfahren gemäß den Ansprüchen 1 bis 9, **dadurch gekennzeichnet, dass** als Kohlenstoffquelle im Wesentlichen Glyzerin und einer oder mehrere der Zucker ausgewählt aus der Gruppe Glukose, Saccharose und Fruktose eingesetzt wird, wobei der Anteil des Glyzerins mindestens 10 % beträgt.

12. Verfahren gemäß einem oder mehreren der Ansprüche 1 bis 11, **dadurch gekennzeichnet, dass** es sich um ein batch-Verfahren, ein fed batch-Verfahren, ein repeated fed batch-Verfahren oder ein kontinuierliches Verfahren handelt.

13. Verfahren gemäß Anspruch 1 bis 12, **dadurch gekennzeichnet, dass** man die gewünschte L-Aminosäure reinigt und isoliert.

14. Verfahren gemäß Anspruch 1 bis 13, **dadurch gekennzeichnet, dass** man ein L-Aminosäure-haltiges Produkt gewinnt, bei dem Bestandteile der Fermentationsbrühe und/oder die Biomasse in ihrer Gesamtheit oder in Anteilen (> 0 bis 100 %) davon im Produkt verbleiben.

## Claims

1. Process for producing L-amino acids, **characterized in that** the following steps are carried out:
1.1 fermentation of L-amino acid-secreting recombinant microorganisms of the *Enterobacteriaceae* family or of the coryneform bacteria that harbour a glpK gene coding for a polypeptide having glycerol kinase activity (ATP-dependent), the amino acid sequence of which is at least 95% identical to that of SEQ ID NO: 2, 4 or 6, said polypeptide being 502 amino acids in length and including in position 428 any proteinogenic amino acid except glycine, in a medium at a desired temperature, wherein the carbon source employed is essentially glycerol or essentially glycerol and one or more of the sugars selected from the group consisting of glucose, sucrose and fructose, and
1.2 accumulation in the fermentation broth of the L-amino acid being produced.

2. Process according to Claim 1, **characterized in that** the proteinogenic amino acid in position 428 is L-glutamic acid, L-aspartic acid, L-isoleucine, L-histidine, L-tryptophan or L-phenylalanine.

3. Process according to Claim 2, **characterized in that** the proteinogenic amino acid in position 428 is L-aspartic acid.

4. Process according to Claims 1 to 3, **characterized in that** the encoded polypeptide comprises the amino acid sequence of SEQ ID NO: 2, SEQ ID NO: 4 or SEQ ID NO: 6 including the said amino acid substitution in position 428, wherein the amino acid sequence additionally comprises one or more of the features selected from the group consisting of one or no more than 10 conservative amino acid substitutions, a C-terminal extension by from one to no more than 20 amino acid (s).

5. Process according to Claims 1 to 4, **characterized in that** the encoded polypeptide comprises the amino acid sequence of SEQ ID NO: 2, SEQ ID NO: 4 or SEQ ID NO: 6 including the said amino acid substitution in position 428.

6. Process according to Claim 4 or 5, **characterized in that** the encoded polypeptide comprises the amino acid sequence of SEQ ID NO: 2 including the said amino acid substitution in position 428.

7. Process according to Claims 1 to 6, **characterized in that** the L-amino acids are produced by fermenting microorganisms of the *Enterobacteriaceae* family, in which additionally at the same time the glpR gene coding for the repressor of the glp regulon is attenuated, more specifically eliminated, or expression is reduced.

8. Process according to Claims 1 to 7, **characterized in that** the L-amino acids are produced by fermenting microorganisms of the *Enterobacteriaceae* family, in which additionally at the same time one or more of the genes selected from the group consisting of:
a) the glpA gene coding for the large subunit of sn-glycerol-3-phosphate dehydrogenase (anaerobic),
b) the glpB gene coding for the membrane-anchoring subunit of sn-glycerol-3-phosphate dehydrogenase (anaerobic),
c) the glpC gene coding for the small subunit of sn-glycerol-3-phosphate dehydrogenase (anaerobic),
d) the glpD gene coding for glycerol-3-phosphate dehydrogenase (aerobic),
e) the glpE gene coding for sulphur transferase,
f) the glpF gene coding for the glycerol facilitator, GlpF,
g) the glpG gene
h) the glpQ gene coding for periplasmic glycerol phosphodiesterase,
i) the glpT gene coding for glycerol-3-phosphate permease,
j) the glpX gene coding for fructose 1,6-bisphosphatase II,
k) the tpiA gene coding for triosephosphate isomerase,
l) the gldA gene coding for glycerol dehydrogenase (NAD-dependent),
m) the dhaK gene coding for the N-terminal domain of dihydroxyacetone kinase,
n) the dhaL gene coding for the C-terminal domain of dihydroxyacetone kinase,
o) the dhaM gene coding for the PTS-protein subunit of dihydroxyacetone kinase,
p) the dhaR gene coding for the activator of the dha operon,
q) the fsa gene coding for fructose-6-phosphate aldolase I, and
r) the talC gene coding for fructose-6-phosphate
aldolase II
are enhanced, more specifically overexpressed.

9. Process according to Claims 1 to 8, **characterized in that** the microorganisms of the *Enterobacteriaceae* family are microorganisms selected from the genera *Escherichia, Erwinia, Providencia* and *Serratia.*

10. Process according to Claims 1 to 9, **characterized in**
**that** the carbon source employed is essentially glycerol.

11. Process according to Claims 1 to 9, **characterized in**
**that** the carbon source employed is essentially glycerol and one or more of the sugars selected from the group consisting of glucose, sucrose and fructose, the proportion of glycerol being at least 10%.

12. Process according to one or more of Claims 1 to 11,
**characterized in that** it is a batch process, a fed-batch process, a repeated fed-batch process or a continuous process.

13. Process according to Claims 1 to 12, **characterized**
**in that** the desired L-amino acid is purified and isolated.

14. Process according to Claims 1 to 13, **characterized**
**in that** an L-amino acid-containing product is obtained in which the product retains fermentation broth components and/or the biomass completely or parts thereof (>0 to 100%).

## Revendications

1. Procédé de préparation d'acides L-aminés, **caractérisé en ce qu'**on met en oeuvre les étapes suivantes :
1.1 fermentation de microorganismes recombinants sécrétant un acide L-aminé, de la famille des Enterobacteriaceae ou des bactéries corynéformes qui contiennent un gène glpK qui code pour un polypeptide ayant une activité de glycérol-kinase (ATP-dépendant), dont la séquence d'acides aminés a une identité d'au moins 95 % avec celle de SEQ ID NO:2, 4 ou 6, le polypeptide ayant une longueur de 502 acides aminés, le polypeptide en position 428 contenant chacun des acides aminés protéinogènes à l'exception de la glycine, dans un milieu à une température souhaitée, lors de laquelle on utilise en tant que source de carbone pour l'essentiel du glycérol ou pour l'essentiel du glycérol et un ou plusieurs des sucres choisis dans le groupe consistant en le glucose, le saccharose et le fructose, et
1.2 accumulation, dans le bouillon de fermentation, de l'acide L-aminé formé.

2. Procédé selon la revendication 1, **caractérisé en ce que** l'acide aminé protéinogène en position 428 est l'acide L-glutamique, l'acide L-asparagique, la L-isoleucine, la L-histidine, le L-tryptophane ou la L-phénylalanine.

3. Procédé selon la revendication 2, **caractérisé en ce que**, pour ce qui concerne l'acide aminé protéinogène en position 428, il s'agit de l'acide L-asparagique.

4. Procédé selon les revendications 1 à 3, **caractérisé en ce que** le polypeptide codé comprend la séquence d'acides aminés de SEQ ID NO:2, SEQ ID NO:4 ou SEQ ID NO:6, y compris l'échange d'acides aminés mentionné en position 428, la séquence d'acides aminés ayant en outre une ou plusieurs des caractéristiques choisies dans le groupe consistant en un ou au plus 10 échanges conservateurs d'acides aminés, une extension au niveau C-terminal d'un à au plus 20 acides aminés.

5. Procédé selon les revendications 1 à 4, **caractérisé en ce que** le polypeptide codé comprend la séquence d'acides aminés de SEQ ID NO:2, SEQ ID NO:4 ou SEQ ID NO:6, y compris l'échange mentionné d'acides aminés en position 428.

6. Procédé selon la revendication 4 ou 5, **caractérisé en ce que** le polypeptide codé comprend la séquence d'acides aminés de SEQ ID NO:2, y compris l'échange mentionné d'acides aminés en position 428.

7. Procédé selon les revendications 1 à 6, **caractérisé en ce que**, pour la préparation des acides L-aminés, on fermente des microorganismes de la famille des Enterobacteriaceae, dans lesquels on atténue le gène glpR codant pour le répresseur du régulon glp, en particulier on l'inactive, ou on diminue l'expression.

8. Procédé selon les revendications 1 à 7, **caractérisé en ce que**, pour la préparation des acides L-aminés, on fermente des microorganismes de la famille des Enterobacteriaceae, dans lesquels on amplifie, en particulier on surexprime en plus et simultanément un ou plusieurs des gènes choisis dans le groupe :
a) le gène glpA, codant pour la grande sous-unité de la sn-glycérol-3-phosphate-déshydrogénase (anaérobie),
b) le gène glpB, codant pour la sous-unité servant à l'ancrage membranaire de la sn-glycérol-3-phosphate-déshydrogénase (anaérobie),
c) le gène glpC, codant pour la petite sous-unité de la sn-glycérol-3-phosphate-déshydrogénase (anaérobie),
d) le gène glpD, codant pour la glycérol-3-phosphate-déshydrogénase (aérobie),
e) le gène glpE, codant pour la soufre-transférase,
f) le gène glpF, codant pour le facilitateur du glycérol GlpF,
g) le gène glpG,
h) le gène glpQ, codant pour la glycérol-phosphodiestérase périplasmique,
i) le gène glpT, codant pour la glycérol-3-phosphate-perméase,
j) le gène glpX, codant pour la fructose-1,6-bisphosphatase II,
k) le gène tpiA, codant pour la triosephosphate-isomérase,
l) le gène gldA, codant pour la glycérol-déshydrogénase (NAD-dépendante),
m) le gène dhaK, codant pour le domaine N-terminal de la dihydroxyacétone-kinase,
n) le gène dhaL, codant pour le domaine C-terminal de la dihydroxyacétone-kinase,
o) le gène dhaM, codant pour la sous-unité protéine PTS de la dihydroxyacétone-kinase,
p) le gène dhaR, codant pour l'activateur de l'opéron dha,
q) le gène fsa, codant pour la fructose-6-phosphate-aldolase I, et
r) le gène talC, codant pour la fructose-6-phosphate-aldolase II.

9. Procédé selon les revendications 1 à 8, **caractérisé en ce que**, pour ce qui concerne les microorganismes de la famille des Enterobacteriaceae, il s'agit d'un microorganisme choisi parmi les genres Escherichia, Erwinia, Providencia et Serratia.

10. Procédé selon les revendications 1 à 9, **caractérisé en ce qu'**on utilise en tant que source de carbone pour l'essentiel du glycérol.

11. Procédé selon les revendications 1 à 9, **caractérisé en ce qu'**on utilise en tant que source de carbone pour l'essentiel du glycérol et un ou plusieurs des sucres choisis dans le groupe consistant en le glucose, le saccharose et le fructose, la proportion du glycérol étant d'au moins 10 %.

12. Procédé selon l'une ou plusieurs des revendications 1 à 11, **caractérisé en ce qu'**il s'agit d'un procédé discontinu, d'un procédé de fermentation discontinue alimentée, d'un procédé discontinu alimenté répété, ou d'un procédé continu.

13. Procédé selon les revendications 1 à 12, **caractérisé en ce qu'**on purifie et isole l'acide L-aminé souhaité.

14. Procédé selon les revendications 1 à 13, **caractérisé en ce qu'**on obtient un produit contenant un acide L-aminé, dans lequel des constituants du bouillon de fermentation et/ou de la biomasse restent dans le produit dans leur totalité ou selon des parties (> 0 à 100 %) de cette dernière.
